(12) ## EUROPÄISCHE PATENTSCHRIFT

(45) Veröffentlichungstag und Bekanntmachung des Hinweises auf die Patenterteilung:
**12.06.2024 Patentblatt 2024/24**

(21) Anmeldenummer: **20165078.5**

(22) Anmeldetag: **24.03.2020**

(51) Internationale Patentklassifikation (IPC):
**A61N 1/362** (2006.01)    **A61N 1/368** (2006.01)
**A61B 5/363** (2021.01)

(52) Gemeinsame Patentklassifikation (CPC):
**A61N 1/3624; A61B 5/363; A61N 1/3688**

(54) **IMPLANTIERBARE ANORDNUNG ZUR STIMULATION EINES MENSCHLICHEN ODER TIERISCHEN HERZENS**

IMPLANTABLE ASSEMBLY FOR STIMULATING A HUMAN OR ANIMAL HEART

DISPOSITIF POUVANT ÊTRE IMPLANTÉ DESTINÉ À LA STIMULATION D'UN C UR HUMAIN OU ANIMAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.04.2019 DE 102019110289**

(43) Veröffentlichungstag der Anmeldung:
**04.11.2020 Patentblatt 2020/45**

(73) Patentinhaber: **BIOTRONIK SE & Co. KG**
**12359 Berlin (DE)**

(72) Erfinder:
• **DÖRR, Thomas**
**12437 Berlin (DE)**
• **ERSHOV, Sergey**
**14057 Berlin (DE)**
• **RADTKE, Torsten**
**12589 Berlin (DE)**
• **RÖMER, Martin**
**14195 Berlin (DE)**
• **WEISS, Ingo**
**12435 Berlin (DE)**

(74) Vertreter: **Biotronik Corporate Services SE**
**Corporate Intellectual Property**
**Sieversufer 7-9**
**12359 Berlin (DE)**

(56) Entgegenhaltungen:
**EP-A2- 2 077 136      WO-A2-2006/105140**
**US-A1- 2003 120 317   US-B2- 8 457 739**

• **TAKAGI M ET AL: "Acute improvement of atrial mechanical stunning after electrical cardioversion of persistent atrial fibrillation: comparison between biatrial and single atrial pacing", HEART, BMJ, LONDON, GB, Bd. 91, Nr. 1, 1. Januar 2005 (2005-01-01) , Seiten 58-63, XP002397041, ISSN: 1355-6037, DOI: 10.1136/HRT.2003.032334**

**Beschreibung**

Die vorliegende Erfindung betrifft eine implantierbare Anordnung zur Stimulation eines menschlichen oder tierischen Herzens gemäß Anspruch 1 und ein zur Steuerung geeignetes Computerprogrammprodukt gemäß Anspruch 11.

Implantierbare Anordnungen zur Stimulation eines menschlichen oder tierischen Herzens, wie etwa Herzschrittmacher, sind seit langem bekannt. Sie können unterschiedliche Funktionen ausführen. Unter anderem ist es bekannt, solche Herzschrittmacher zur Behandlung von Vorhof-Rhythmusstörungen einzusetzen. Dabei können verschiedene Stimulationsprogramme von einem entsprechenden Herzschrittmacher durchgeführt werden, um das behandelte Herz wieder in einen normalen Zustand zu bringen.

Häufig ist es schwierig vorherzusagen, welche Behandlungsart sinnvoll ist, um eine Rhythmusstörung, insbesondere eine tachykarde Vorhof-Rhythmusstörung, adäquat und effektiv zu behandeln.

Die US 6,876,880 B2 beschreibt beispielsweise die Möglichkeit, dass nacheinander unterschiedliche atriale Stimulationen erfolgen. Ferner wird in jenem US-Patent beschrieben, dass der Behandlungserfolg von dem Zustand des Herzens abhängt. Daher eröffnet jenes US-Patent die Möglichkeit, bereits erfolglos durchgeführte atriale Stimulationen erneut durchzuführen, wenn eine bestimmte Zeit verstrichen ist oder sich der Zustand des Herzens anderweitig verändert hat. Auf diesem Weg soll eine verbesserte Behandlung atrialer Rhythmusstörungen erreicht werden. Der Erfolg dieses Vorgehens ist jedoch nur mäßig, da man letztlich auf eine spontane Änderung des Zustands des Herzens bzw. auf ein Verstreichen einer ausreichend großen Zeit angewiesen ist.

Die US 7,107,098 B2 beschreibt ein Verfahren und eine Vorrichtung zur Erzeugung und Auswahl von Therapien oder Therapiehierarchien, die zur Behandlung atrialer oder ventrikulärer Tachykardien geeignet sind. Dabei ist vorgesehen, eine in der Vergangenheit erreichte Effektivität einer Therapie zu speichern und dieser Therapie dann eine höhere Hierarchie zuzuordnen.

Die US 7,280,869 B2 beschreibt ein Verfahren zur Bestimmung, ob eine tachykarde Arrhythmie beendet wurde. Dabei wird ein Herzschlagmuster, das eine Sequenz von atrialen und ventrikulären Depolarisationen repräsentiert, für diese Bestimmung herangezogen.

Die US 8 457 739 B2 beschreibt ein Verfahren und eine Vorrichtung zur Steuerung einer atrialen Overdrive-Stimulationstherapie. Das Verfahren umfasst die Erfassung einer atrialen Arrhythmie-Episode und die Bestimmung, ob die atriale Arrhythmie-Episode eine frühe wiederkehrende Episode ist. Die Abgabe der atrialen Overdrive-Stimulationstherapie wird als Reaktion auf die frühe wiederkehrende Episode aktiviert und beginnt mit der Erfassung einer atrialen Arrhythmie-Episode oder einer langen Pause.

Die EP 2 077 136 A2 beschreibt ein System und ein Verfahren zur Bereitstellung von Stimulationsimpulsen nach einem Kardioversions-/Defibrillationsschock, wobei die Stimulationsimpulse eine Stimulationsrate mit einem Anfangswert aufweisen. Die Stimulationsrate wird vom Anfangswert aus verringert, bis mindestens eine intrinsische Herzkontraktion festgestellt wird. In einer Ausführungsform wird die Stimulationsfrequenz nach einer bestimmten Anzahl von Herzzyklen um einen bestimmten Betrag verringert. Die Abgabe der festgelegten Anzahl von Stimulationsimpulsen und die Verringerung der Stimulationsrate um den festgelegten Betrag wird dann so lange wiederholt, bis mindestens eine intrinsische Herzkontraktion festgestellt wird. Aus der mindestens einen intrinsischen Herzkontraktion wird dann eine intrinsische Herzfrequenz bestimmt. Die Stimulationsfrequenz wird dann erhöht und so gehalten, dass sie über der intrinsischen Herzfrequenz liegt (d. h. größer ist als diese).

Die WO 2006/105140 A2 beschreibt eine implantierbare medizinische Vorrichtung (IMD), welche einen Therapieschaltkreis zur Abgabe einer atrialen Stimulation und einen Steuerschaltkreis zum Erkennen einer Rückkehr zum Sinusrhythmus, zum Bestimmen der Dauer der atrialen Arrhythmie, die der Rückkehr zum Sinusrhythmus vorausgeht, und zum Steuern des Therapieschaltkreises zur Abgabe einer vorübergehenden atrialen Stimulation auf der Grundlage der Dauer umfasst. Ferner umfasst das implantierbare medizinische Gerät Mittel zur Verwendung von Daten von einem hämodynamischen Sensor, um die Stimulationsrate der vorübergehenden atrialen Stimulation auszuwählen. Die Stimulation kann eine Overdrive-Stimulation oder eine gepaarte Stimulation sein.

Selbst wenn eine bestimmte Behandlungsart ausgewählt wurde, ist ein Therapieerfolg der durchgeführten Behandlung häufig nicht langfristig anhaltend. Vielmehr kann das behandelte Herz schon nach einer kurzen Zeitspanne wieder in einen Zustand mit atrialer Tachykardie zurückfallen.

Der vorliegenden Erfindung liegt die Aufgabe zugrunde, einen Herzschrittmacher bereitzustellen, der eine nachhaltigere Behandlung tachykarder Vorhof-Rhythmusstörungen ermöglicht, um die Therapieeffizienz derartiger Behandlungen zu erhöhen.

Diese Aufgabe wird durch eine implantierbare Anordnung zur Stimulation eines menschlichen oder tierischen Herzens mit den Merkmalen des Anspruchs 1 gelöst. Eine solche Anordnung ist insbesondere zur dauerhaften Implantation in einem menschlichen oder tierischen Patienten vorgesehen. Wenn die Anordnung in einem tierischen Patienten implantiert werden soll, handelt es sich bei dem Patienten vorzugsweise um ein Säugetier, beispielsweise um ein Säugetier, das ausgewählt ist aus der Gruppe bestehend aus Nagetieren, Pferden, Hunden und Katzen.

Die Anordnung weist einen Prozessor, eine Speichereinrichtung, eine atriale Stimulationseinrichtung und eine Detektionseinrichtung zum Detektieren einer atrialen Tachykardie auf.

Erfindungsgemäß ist vorgesehen, dass die Speichereinrichtung ein computerlesbares Programm aufweist, das den Prozessor dazu veranlasst, die nachfolgend erläuterten Schritte auszuführen, wenn das Programm auf dem Prozessor ausgeführt wird.

Zunächst wird mittels der Detektionseinrichtung detektiert, ob in einem menschlichen oder tierischen Herz eine zu behandelnde atriale Tachykardie vorliegt.

Wenn eine solche zu behandelnde atriale Tachykardie detektiert wurde, wird mittels der atrialen Stimulationseinrichtung eine atriale anti-tachykarde Stimulation ausgeführt.

Nach dem Ausführen der atrialen anti-tachykarden Stimulation und nur, wenn keine tachykarde intrinsische atriale Aktivität vorliegt, wird eine atriale Nachbehandlungsstimulation ausgeführt. Hierzu wird insbesondere ebenfalls die atriale Stimulationseinrichtung verwendet. Durch die Nachbehandlungsstimulation wird eine Veränderung des kardialen Zustandes bewirkt, wodurch die langfristige Therapieerfolgsrate der atrialen Überstimulation, die im Rahmen der atrialen anti-tachykarden Stimulation durchgeführt wurde, erhöht wird. Durch die atriale Nachbehandlung wird insgesamt ein nachhaltiger Effekt der atrialen anti-tachykarden Stimulation erreicht. Denn das behandelte Herz neigt durch die atriale Nachbehandlung weniger stark dazu, erneut in einen Zustand atrialer Tachykardie zu verfallen.

In einer Variante veranlasst das Programm den Prozessor dazu, nach dem Ausführen der atrialen anti-tachykarden Stimulation zunächst mittels der Detektionseinrichtung zu überprüfen, ob die atriale Tachykardie durch die durchgeführte Stimulation beendet werden konnte. Es erfolgt also eine Terminierungsdetektion der atrialen Tachykardie. Nur wenn die Beendigung der atrialen Tachykardie positiv ermittelt wurde, wird die atriale Nachbehandlungsstimulation ausgeführt. Dadurch wird erreicht, dass die atriale Nachbehandlungsstimulation zu einem Zeitpunkt durchgeführt wird, in dem das behandelte Herz keine atriale anti-tachykarde Stimulation mehr benötigt, damit die zuvor detektierte atriale Tachykardie beendet werden kann.

In einer Variante veranlasst das Programm den Prozessor dazu, die atriale Nachbehandlungsstimulation als atriale Überstimulation auszuführen. Dabei grenzt sich diese atriale Überstimulation von der im Rahmen der atrialen anti-tachykarden Überstimulation dadurch ab, dass sie erstens längerfristiger als eine atriale anti-tachykarde Überstimulation angelegt ist, d.h. im Bereich von Minuten bis zu Tagen. Gemäß einer Ausführungsform ist die atriale Nachbehandlung im Bereich von bis zu 7 Tagen, 6 Tagen, 5 Tagen, 4 Tagen, 3 Tagen, 2 Tagen, oder 1 Tag ausgelegt. Gemäß weiterer Ausführungsformen ist die atriale Nachbehandlung im Bereich bis zu 120 Minuten, 60 Minuten, 30 Minuten, 20 Minuten, 10 Minuten, 5 Minuten, 4 Minuten, 3 Minuten, 2 Minuten, 1 Minute, oder 0,5 Minuten ausgelegt. Des Weiteren unterscheidet sich die erfindungsgemäße atriale Nachbehandlungsstimulation von der bekannten anti-tachykarden Überstimulation dadurch, dass sie nur abgerufen wird, wenn keine tachykarde intrinsische (herz-eigene) atriale Aktivität vorliegt, d.h. zum Beispiel wenn der atriale intrinsische Herzrhythmus unter 100 bpm liegt. Gemäß einer besonders vorteilhaften Ausführungsform der Erfindung wird die atriale Nachbehandlungsstimulation mit einer Stimulationsfrequenz von kleiner gleich 200 bpm, 180 bpm, 150 bpm, 140 bpm, 130 bpm, 120 bpm, 110 bpm, oder 100 bpm abgegeben. Im Vergleich werden bei der bekannten anti-tachykarden Überstimulation Stimulationsfrequenzen von 375 bpm, oder im Fall von Hochfrequenz-Bursts, Frequenzen von bis zu 3000 bpm (50 Hz) angewandt.

In einer Variante wird die atriale Nachbehandlungsstimulation als rechtsatriale Overdrive-Stimulation ausgeführt. Bei einer derartigen Overdrive-Stimulation wird das entsprechende Atrium mit einer Frequenz stimuliert, die höher als die gewöhnliche (intrinsische) nicht tachykarde atriale Frequenz ist. Allerdings ist die Stimulationsfrequenz bei einer Overdrive-Stimulation geringer als bei einer atrialen anti-tachykarden Stimulation (z.B. kleiner gleich 130 bpm).

In einer weiteren Variante veranlasst das Programm den Prozessor dazu, die atriale Nachbehandlungsstimulation als linksatriale Overdrive-Stimulation auszuführen. Die atriale Nachbehandlungsstimulation kann also gezielt die Frequenz des atrialen Rhythmus eines einzigen Atriums beeinflussen, insbesondere die Rückkehr von einem tachykarden Zustand in einen Normalzustand verzögern.

In einer weiteren Variante ist vorgesehen, dass die atriale Nachbehandlungsstimulation als biatriale Overdrive-Stimulation ausgestaltet ist. In einem solchen Fall veranlasst das Programm den Prozessor dazu, die atriale Frequenz des Rhythmus beider Atrien zu erhöhen.

Durch eine derartige Overdrive-Nachbehandlung eines oder beider Atrien wird das Herz nach der atrialen anti-tachykarden Stimulation wieder langsamer in den Normalzustand überführt, als wenn keine Nachbehandlungsstimulation durchgeführt würde. Es wird also ein sanfter Übergang zwischen der atrialen anti-tachykarden Stimulation und dem normalen Herzrhythmus erreicht, der eine schonende Behandlung des Herzens bewirkt und für einen nachhaltigeren Effekt der atrialen anti-tachykarden Stimulation sorgt.

In einer Variante veranlasst das Programm den Prozessor dazu, die atriale Nachbehandlungsstimulation während einer ersten Zeitdauer auszuführen. Dabei ist diese erste Zeitdauer vorgebbar. Es handelt sich mithin um eine definierte Zeitspanne in einem Bereich von 1 Minute bis 7 Tagen.

In einer Variante wird die atriale Nachbehandlungsstimulation nicht für eine (fixe) Zeitspanne durchgeführt, sondern orientiert sich an dem Herzrhythmus des behandelten Patienten. So veranlasst das Programm den Prozessor in dieser Variante dazu, die atriale Nachbehandlungsstimulation während einer definierten Anzahl an Herzzyklen auszuführen. In dieser Variante kann noch individueller auf die unterschiedlichen Herzrhythmen der einzelnen Patienten eingegangen werden, was den Nachhaltigkeitserfolg der Nachbehandlungsstimulation weiter erhöhen kann.

In einer Variante veranlasst das Programm den Prozessor dazu, die atriale Nachbehandlungsstimulation in einer Art und Weise auszuführen, um ein bestimmtes physiologisches Ziel zu erreichen. So kann die Nachbehandlungsstimulation beispielsweise derart ausgeführt werden, dass der Druck im linken und/oder rechten Atrium zumindest kurzzeitig gesenkt wird. Alternativ oder zusätzlich kann die Nachbehandlungsstimulation derart ausgeführt werden, dass das Risiko einer Mitralklappenregurgitation zumindest kurzzeitig reduziert oder gänzlich vermieden wird. Alternativ oder zusätzlich kann die Nachbehandlungsstimulation derart ausgeführt werden, dass die Vorlast des Herzens zumindest kurzzeitig reduziert wird.

Alternativ oder zusätzlich kann die Nachbehandlungsstimulation derart ausgeführt werden, dass der Blutdruck eines Patienten, dessen Herz durch die implantierbare Anordnung stimuliert wird, zumindest kurzzeitig gesenkt wird. Alternativ oder zusätzlich kann die Nachbehandlungsstimulation schließlich derart ausgeführt werden, dass eine myokardiale Sauerstoffbilanz zumindest kurzzeitig verbessert wird. Durch all diese Effekte - entweder einzeln oder in beliebiger Kombination - wird eine Veränderung des kardialen Status erreicht, der die Nachhaltigkeit der zuvor durchgeführten atrialen anti-tachykarden Stimulation erhöht und somit den Therapieerfolg der atrialen anti-tachykarden Stimulation verbessert.

In einer Variante ist die atriale Stimulationseinrichtung dazu ausgelegt, die atriale anti-tachykarde Stimulation und/oder die atriale Nachbehandlungsstimulation als elektrische Stimulation oder als optische Stimulation auszuführen. Somit kann nicht nur die atriale anti-tachykarde Stimulation unter Anwendung unterschiedlicher physikalischer Wirkprinzipien durchgeführt werden, sondern auch die atriale Nachbehandlungsstimulation. Die unterschiedlichen zur Verfügung stehenden Behandlungsvarianten lassen sich dabei insbesondere unter Berücksichtigung der Schwere der detektierten atrialen Tachykardie und der sich daraus ergebenden sinnvollerweise durchzuführenden Nachbehandlungsstimulation auswählen.

Ein Aspekt, der jedoch nicht beansprucht wird, betrifft ein Verfahren zur Steuerung des Betriebs einer implantierbaren Anordnung zur Stimulation eines menschlichen oder tierischen Herzens. Dabei eignet sich dieses Verfahren insbesondere zur Steuerung des Betriebs einer implantierbaren Anordnung gemäß den vorherigen Erläuterungen. Dieses Steuerungsverfahren weist die nachfolgend erläuterten Schritte auf.

Zunächst wird mittels einer Detektionsvorrichtung erfasst, ob in einem menschlichen oder tierischen Herz eine zu behandelnde atriale Tachykardie vorliegt.

Wenn eine derartige zu behandelnde atriale Tachykardie detektiert wurde, wird in einer atrialen Stimulationseinrichtung mindestens ein Impuls für eine atriale anti-tachykarde Stimulation erzeugt.

Nach der Erzeugung des Impulses für die atriale anti-tachykarde Stimulation wird in der atrialen Stimulationseinrichtung mindestens ein Impuls für eine atriale Nachbehandlungsstimulation erzeugt. Dabei kann zuvor überprüft werden, ob in dem menschlichen oder tierischen Herz immer noch eine zu behandelnde atriale Tachykardie vorliegt. Die Erzeugung des mindestens einen Impulses für die atriale Nachbehandlungsstimulation kann davon abhängig gemacht werden, ob eine Terminierung einer zuvor detektierten behandlungspflichtigen atrialen Tachykardie ermittelt wurde. Hierzu kann ein entsprechendes Detektorsignal als Eingangsgröße für das Steuerungsverfahren dienen.

Ein Aspekt der vorliegenden Erfindung betrifft ein Computerprogrammprodukt mit einem computerlesbaren Code, der einen Prozessor dazu veranlasst, die nachfolgend erläuterten Schritte auszuführen, wenn der Code auf dem Prozessor ausgeführt wird.

Zunächst wird mittels einer Detektionsvorrichtung erfasst, ob in einem menschlichen oder tierischen Herz eine zu behandelnde atriale Tachykardie vorliegt.

Wenn eine solche zu behandelnde atriale Tachykardie detektiert wurde, wird mittels einer atrialen Stimulationseinrichtung eine atriale anti-tachykarde Stimulation ausgeführt.

Nach dem Ausführen der atrialen anti-tachykarden Stimulation und nur, wenn keine tachykarde intrinsische atriale Aktivität vorliegt, wird, insbesondere ebenfalls mittels der atrialen Stimulationseinrichtung, eine atriale Nachbehandlungsstimulation ausgeführt.

Ein Aspekt, der jedoch nicht beansprucht wird, betrifft ein Verfahren zur Behandlung eines menschlichen oder tierischen Patienten, der eine solche Behandlung benötigt. Dabei wird eine implantierbare Anordnung zur Stimulation des Herzens des Patienten eingesetzt. Diese Anordnung weist einen Prozessor, eine Speichereinrichtung, eine atriale Stimulationseinrichtung und eine Detektionseinrichtung zum Detektieren einer atrialen Tachykardie auf. Das Behandlungsverfahren weist die nachfolgend erläuterten Schritte auf.

Zunächst wird mittels der Detektionseinrichtung überprüft, ob in dem Herzen des Patienten eine zu behandelnde atriale Tachykardie vorliegt.

Wenn eine derartige atriale Tachykardie detektiert wurde, wird mittels der atrialen Stimulationseinrichtung eine atriale anti-tachykarde Stimulation durchgeführt.

Nach dem Durchführen der atrialen anti-tachykarden Stimulation wird eine atriale Nachbehandlungsstimulation durchgeführt. Durch diese atriale Nachbehandlungsstimulation wird das Herz des Patienten langsamer in einen Normzustand mit einem gewöhnlichen Herzrhythmus überführt, als wenn die atriale anti-tachykarde Stimulation abrupt beendet würde. Durch diesen sanfteren Übergang zu einem gewöhnlichen Herzrhythmus wird ein nachhaltiger und länger anhaltender Therapieeffekt der durchgeführten atrialen anti-tachykarden Stimulation erreicht.

Ein Aspekt der vorliegenden Erfindung betrifft eine implantierbare Anordnung zur Stimulation eines menschlichen oder tierischen Herzens mit den nachfolgend erläuterten Merkmalen. Eine solche Anordnung ist insbesondere zur dauerhaften Implantation in einem menschlichen oder tierischen Patienten vorgesehen. Wenn die Anordnung in einem tierischen Patienten implantiert werden soll, handelt es sich bei dem Patienten vorzugsweise um ein Säugetier, beispielsweise um ein Säugetier, das ausgewählt ist aus der Gruppe bestehend aus Nagetieren, Pferden, Hunden und Katzen.

Die Anordnung weist einen Prozessor, eine Speichereinrichtung, eine atriale Stimulationseinrichtung, eine ventrikuläre Stimulationseinrichtung und eine erste Detektionseinrichtung auf. Die erste Detektionseinrichtung dient zum Detektieren einer atrialen Tachykardie, also einem Schlagen des Vorhofs des Herzens mit einer abnormal erhöhten Frequenz.

Erfindungsgemäß zeichnet sich die Anordnung dadurch aus, dass die Speichereinrichtung ein computerlesbares Programm aufweist, das den Prozessor dazu veranlasst, die nachfolgend erläuterten Schritte auszuführen, wenn es auf dem Prozessor ausgeführt wird.

Zunächst wird mittels der ersten Detektionseinrichtung erfasst, ob in einem menschlichen oder tierischen Herzen des Patienten, dem die implantierbare Anordnung implantiert wurde, eine zu behandelnde atriale Tachykardie vorliegt. Eine solche Tachykardie, die auch als behandlungspflichtige Tachykardie bezeichnet werden kann, zeichnet sich dadurch aus, dass der atriale Rhythmus eine kritische Frequenz überschreitet und/oder der resultierende ventrikuläre Rhythmus eine kritische Frequenz über- oder unterschreitet und/oder der atriale Rhythmus als instabil klassifiziert wird und/oder der resultierende ventrikuläre Rhythmus nennenswerte Frequenzschwankungen aufweisen.

Der Begriff der behandlungspflichtigen atrialen Tachykardie umfasst neben einer nichtpathologischen atrialen Tachykardie auch eine pathologische atriale Tachykardie, die auch unter der Bezeichnung atriale Fibrillation oder Vorhofflimmern bekannt ist. In einer Variante bezieht sich das von dem Prozessor durchgeführte Verfahren nur auf die nichtpathologische atriale Tachykardie oder nur auf die pathologische atriale Tachykardie.

Wenn eine zu behandelnde atriale Tachykardie vorliegt, wird mittels der ventrikulären Stimulationseinrichtung eine ventrikuläre Konditionierungsstimulation ausgeführt. Durch diese ventrikuläre Konditionierungsstimulation wird das Herz in einen Zustand überführt, in dem es empfänglicher für eine nachfolgend durchgeführte atriale Stimulation ist. Das Herz wird also ganz gezielt in einen Zustand überführt, indem es eine erhöhte Suszeptibilität gegenüber nachfolgenden Behandlungen aufweist. Folglich wird mit der erfindungsgemäß beanspruchten Anordnung nicht darauf gewartet, dass das Herz von selbst oder durch vorherige atriale Behandlungen in einen anderen Zustand überführt wird. Vielmehr wird eine aktive Veränderung des kardialen Status mittels einer ventrikulären Konditionierungsstimulation herbeigeführt. Dabei ist eine Vielzahl an sich bekannter Konditionierungsstimulationen möglich, um eine derartige Änderung des kardialen Status zu erreichen.

Während und/oder nach der Ausführung der ventrikulären Konditionierungsstimulation wird eine atriale anti-tachykarde Stimulation durchgeführt. Hierfür wird die atriale Stimulationseinrichtung verwendet. Eine derartige atriale anti-tachykarde Stimulation ist auch unter dem Fachbegriff atriale ATP bekannt (die Abkürzung ATP steht für den englischen Fachbegriff "antitachycardia pacing"). Die ATP wird mitunter auch als anti-tachykarde Schrittmachertherapie bezeichnet.

Da das Herz durch die gleichzeitige bzw. vorherige ventrikuläre Konditionierungsstimulation der durchgeführten atrialen ATP weitaus suszeptibler gegenübersteht als ohne ventrikuläre Konditionierungsstimulation, kann der Therapieerfolg der atrialen ATP erhöht werden. Dadurch ist es möglich, dass das Herz des behandelten Patienten wieder schneller in einem normalen Rhythmus schlägt und pathologische Folgen der atrialen Tachykardie wie etwa Leistungseinbrüche, Schwindel und Thrombenbildung weitaus seltener auftreten.

In einer Variante erfolgt die atriale anti-tachykarde Stimulation nur während der Ausführung der ventrikulären Konditionierungsstimulation. In einer weiteren Variante erfolgt die atriale anti-tachykarde Stimulation nur nach der Ausführung der ventrikulären Konditionierungsstimulation. In einer weiteren Variante erfolgt die atriale anti-tachykarde Stimulation sowohl während als auch nach der Ausführung der ventrikulären Konditionierungsstimulation.

In einer Variante ist die atriale Stimulationseinrichtung dazu ausgelegt, die atriale anti-tachykarde Stimulation als elektrische Stimulation oder als optische Stimulation auszuführen. Somit bietet sie unterschiedliche Möglichkeiten, die detektierte atriale Tachykardie zu behandeln. Dabei können unterschiedliche Behandlungsvarianten insbesondere unter Berücksichtigung der Schwere der detektierten atrialen Tachykardie ausgewählt werden.

In einer Variante veranlasst das Programm den Prozessor dazu, die ventrikuläre Konditionierungsstimulation als rechtsventrikuläre Overdrive-Stimulation auszuführen. Bei einer Overdrive-Stimulation wird der entsprechende Ventrikel mit einer Frequenz stimuliert, die höher als die gewöhnliche (intrinsische) Ventrikelfrequenz ist. Dadurch kann eine Erhöhung der Frequenz des ventrikulären Rhythmus erreicht werden.

In einer anderen Variante veranlasst das Programm den Prozessor dazu, die ventrikuläre Konditionierungsstimulation als linksventrikuläre Overdrive-Stimulation auszuführen. Die ventrikuläre Konditionierungsstimulation kann also gezielt lediglich die Frequenz des ventrikulären Rhythmus eines einzigen Ventrikels beeinflussen, insbesondere erhöhen.

In einer weiteren Variante ist es vorgesehen, dass das Programm den Prozessor dazu veranlasst, beide Ventrikel gleichermaßen zu stimulieren. In diesem Fall wird die ventrikuläre Konditionierungsstimulation als biventrikuläre Overdrive-Stimulation ausgeführt. Auf diese Weise kann eine gleichzeitige Erhöhung der Frequenz des intrinsischen ventrikulären Rhythmus beider Ventrikel erreicht werden.

In einer Variante veranlasst das Programm den Prozessor dazu, die ventrikuläre Konditionierungsstimulation als biventrikuläre Overdrive-Stimulation auszuführen und dabei eine von einer regulären Stimulation abweichende VV-Zeit vorzugeben. Die VV-Zeit (auch als VV-Delay bezeichnet) gibt die Zeit an, die zwischen der Stimulation des rechten und des linken Ventrikels verstreicht. Sie wird auch als intraventrikuläre Verzögerungszeit bezeichnet. Wenn beide Ventrikel zur gleichen Zeit stimuliert werden, beträgt die VV-Zeit Null. Typischerweise ist sie jedoch größer als Null, um eine spätere Stimulation des linken Ventrikels als des rechten Ventrikels zu erreichen. Wenn eine von einer regulären Stimulation abweichende VV-Zeit gewählt wird, kommt es zu einer Veränderung des kardialen Status, wodurch das Herz gegenüber einer gleichzeitig oder nachfolgend durchgeführten atrialen anti-tachykarden Stimulation suszeptibler ist.

In einer Variante veranlasst das Programm den Prozessor dazu, die ventrikuläre Konditionierungsstimulation derart auszuführen, dass ventrikuläre Pausen kompensiert werden. Dies lässt sich beispielsweise durch eine Stärke der ventrikulären Konditionierungsstimulation und/oder eine Einstellung der VV-Zeit und/oder der Abgabe zusätzlicher ventrikulärer Stimuli und/oder der Einstellung alternativer ventrikulärer Stimulationsvektoren (z.B. zwischen rechts- und linksventrikulärer Elektrode) erreichen.

In einer Variante veranlasst das Programm den Prozessor dazu, die ventrikuläre Konditionierungsstimulation mit einer kurzen AV-Zeit an die detektierte atriale Tachykardie anzukoppeln. Dabei wird ein Verhältnis von n: 1 vorgegeben. Konkret veranlasst das Programm den Prozessor dazu, die ventrikuläre Konditionierungsstimulation mit einer AV-Zeit von 10 bis 110 ms an detektierte Aktionen der atrialen Tachykardie in einem Verhältnis n: 1 anzukoppeln, wobei n der Anzahl detektierter Aktionen der atrialen Tachykardie entspricht und n 2 bis 6 beträgt. Die AV-Zeit (auch als AV-Delay bezeichnet) gibt die Zeit an, die zwischen einer Stimulation des rechten Atriums und des rechten Ventrikels verstreicht. Sie wird auch als atrio-ventrikuläre Verzögerungszeit bezeichnet. Wenn diese AV-Zeit möglichst gering gehalten wird und zudem die ventrikuläre Konditionierungsstimulation an die detektierte atriale Tachykardie angepasst wird, kann eine besonders vorteilhafte Reaktion des derart behandelten Herzens auf die ventrikuläre Konditionierungsstimulation erwartet werden, sodass eine gleichzeitige oder nachfolgende atriale anti-tachykarde Stimulation besonders effektiv durchgeführt werden kann.

In einer Variante veranlasst das Programm den Prozessor dazu, die ventrikuläre Konditionierungsstimulation in einer Art und Weise auszuführen, um ein bestimmtes physiologisches Ziel zu erreichen. So kann die Konditionierungsstimulation beispielsweise derart ausgeführt werden, dass der Druck im linken und/oder rechten Atrium zumindest kurzzeitig gesenkt wird. Alternativ oder zusätzlich kann die Konditionierungsstimulation derart ausgeführt werden, dass das Risiko einer Mitralklappenregurgitation zumindest kurzzeitig reduziert oder gänzlich vermieden wird. Alternativ oder zusätzlich kann die Konditionierungsstimulation derart ausgeführt werden, dass die Vorlast des Herzens zumindest kurzzeitig reduziert wird. Alternativ oder zusätzlich kann die Konditionierungsstimulation derart ausgeführt werden, dass der Blutdruck eines Patienten, dessen Herz durch die implantierbare Anordnung stimuliert wird, zumindest kurzzeitig gesenkt wird. Alternativ oder zusätzlich kann die Konditionierungsstimulation schließlich derart ausgeführt werden, dass eine myokardiale Sauerstoffbilanz zumindest kurzzeitig verbessert wird. Durch all diese Effekte - entweder einzeln oder in beliebiger Kombination - wird eine Veränderung des kardialen Status erreicht, der eine Suszeptibilität des Herzens für eine gleichzeitig oder nachfolgend stattfindende atriale anti-tachykarde Stimulation erhöht.

In einer Variante weist die implantierbare Anordnung eine zweite Detektionseinrichtung auf, die zum Detektieren eines Effects der ventrikulären Konditionierungsstimulation auf die atriale anti-tachykarde Stimulation dient. Durch diese zweite Detektionseinrichtung, die auch Teile der ersten Detektionseinrichtung umfassen kann, ist es also möglich, den durch die ventrikuläre Konditionierungsstimulation erreichten kardialen Effekt mittelbar oder unmittelbar (also direkt oder indirekt) zu erfassen. Dadurch kann die Effektivität der von der implantierbaren Anordnung ausgeführten Stimulation insgesamt verbessert werden. Denn auf diese Weise ist es möglich, eine positive Rückmeldung über den erzielten Effekt in Bezug auf den kardialen Status zu erhalten. Die zweite Detektionseinrichtung kann beispielsweise dieselbe Elektrode nutzen, die auch die erste Detektionseinrichtung nutzt. Es ist also grundsätzlich denkbar, dass die zweite Detektionseinrichtung den Effekt der ventrikulären Konditionierungsstimulation im Atrium des behandelten Herzens erfasst.

In einer Variante veranlasst das Programm den Prozessor dazu, einen Erfolg der atrialen anti-tachykarden Stimulation und die im Zusammenhang mit dieser Stimulation durchgeführte ventrikuläre Konditionierungsstimulation zu speichern. Auf diese Weise können Wertepaare gebildet werden, die jeweils den Erfolg der atrialen anti-tachykarden Stimulation und die währenddessen oder zuvor durchgeführte ventrikuläre Konditionierungsstimulation umfassen. Für eine nachfolgende Behandlung des Herzens kann dann mindestens ein Wertepaar aus den gespeicherten Wertepaaren ausgewählt werden. Beispielsweise kann das Wertepaar ausgewählt werden, bei dem der beste Erfolg der atrialen anti-tachykarden Stimulation erzielt wurde. In gleicher Weise kann beispielsweise ein Wertepaar ausgeschlossen werden, bei dem trotz ventrikulärer Konditionierungsstimulation nicht der erwünschte Erfolg der atrialen anti-tachykarden Stimulation erreicht werden konnte. Die Speicherung kann dabei in der Speichereinrichtung der implantierbaren Anordnung erfolgen. Alternativ kann noch eine weitere Speichereinrichtung in der implantierbare Anordnung vorgesehen sein, die zum Abspeichern der Wertepaare eingesetzt wird.

Ein Aspekt, der jedoch nicht beansprucht wird, betrifft ein Verfahren zur Steuerung des Betriebs der implantierbaren Anordnung zur Stimulation eines menschlichen oder tierischen Herzens. Dieses Verfahren weist die nachfolgend erläuterten Schritte auf.

Zunächst wird mittels einer ersten Detektionseinrichtung erfasst, ob in einem menschlichen oder tierischen Herz eine zu behandelnde (behandlungspflichtige) atriale Tachykardie vorliegt.

Wenn dies der Fall ist, wird mindestens ein Impuls für eine ventrikuläre Konditionierungsstimulation in einer ventrikulären Stimulationseinrichtung erzeugt. Statt eines einzelnen Pulses kann auch eine Pulsfolge aus Pulsen mit gleicher oder unterschiedlicher Amplitude und gleichbleibender, ansteigender oder absteigende Frequenz ausgewählt werden.

Zeitgleich mit und/oder nach der Erzeugung des mindestens einen Pulses in der ventrikulären Stimulationseinrichtung wird mindestens ein Puls für eine atriale anti-tachykarde Stimulation in einer atrialen Stimulationseinrichtung erzeugt. Beispielsweise kann es sich bei diesem mindestens einen Puls ebenfalls um eine Pulsfolge von verschiedenen Pulsen mit gleicher oder unterschiedlich große Amplitude handeln, wobei die Frequenz zwischen den einzelnen Pulsen gleichbleibend, ansteigend oder abfallend ausgestaltet sein kann. Dieses Verfahren bezieht sich also auf die Erzeugung von mindestens einem ventrikulären Konditionierungsstimulationspuls und mindestens einem atrialen Stimulationspuls innerhalb der implantierbaren Anordnung.

Ein Aspekt der vorliegenden Erfindung betrifft ein nicht-flüchtiges Computerprogrammprodukt mit einem computerlesbaren Code, der einen Prozessor dazu veranlasst, die nachfolgend erläuterten Schritte auszuführen, wenn er auf dem Prozessor ausgeführt wird. Zunächst wird mittels einer ersten Detektionseinrichtung erfasst, ob in einem menschlichen oder tierischen Herz eine zu behandelnde atriale Tachykardie vorliegt.

Wenn eine zu behandelnde atriale Tachykardie vorliegt, wird mittels einer ventrikulären Stimulationseinrichtung eine ventrikuläre Konditionierungsstimulation ausgeführt.

Während und/oder nach der Ausführung der ventrikulären Konditionierungsstimulation wird mittels einer atrialen Stimulationseinrichtung eine atriale anti-tachykarde Stimulation ausgeführt.

Ein Aspekt, der jedoch nicht beansprucht wird, betrifft ein Verfahren zur Behandlung eines menschlichen oder tierischen Patienten, die eine solche Behandlung benötigt. Diese Behandlung erfolgt mittels einer implantierbaren Anordnung zur Stimulation des Herzens des Patienten. Dabei weist die Anordnung einen Prozessor, eine Speichereinrichtung, eine atriale Stimulationseinrichtung, eine ventrikuläre Stimulationseinrichtung sowie eine erste Detektionseinrichtung zum Detektieren einer atrialen Tachykardie auf. Das Verfahren umfasst die nachfolgend erläuterten Schritte.

Zunächst wird mittels der ersten Detektionsvorrichtung erfasst, ob in dem Herz eine zu behandelnde atriale Tachykardie vorliegt.

Wenn eine derartige behandlungspflichtige atriale Tachykardie detektiert wurde, wird eine ventrikuläre Konditionierungsstimulation mittels der ventrikulären Stimulationseinrichtung durchgeführt.

Während oder nach der ventrikulären Konditionierungsstimulation wird eine atriale anti-tachykarde Stimulation mittels der atrialen Stimulationseinrichtung durchgeführt. Durch dieses Verfahren wird also ein unmittelbarer therapeutischer Effekt auf das behandelte menschliche oder tierische Herz genommen, um eine detektierte atriale Tachykardie zu behandeln und das behandelte Herz, insbesondere das Atrium des behandelten Herzens, wieder in einen gewöhnlichen Rhythmus zu überführen.

Ein Aspekt der vorliegenden Erfindung betrifft eine implantierbare Anordnung zur Behandlung eines menschlichen oder tierischen Herzens mit den nachfolgend erläuterten Merkmalen. Eine solche Anordnung weist einen Prozessor, eine Speichereinrichtung, eine Behandlungseinrichtung mit einer Behandlungselektrode sowie eine Detektionseinrichtung zum Detektieren eines behandlungsbedürftigen kardialen Ereignisses auf. Die Anordnung zeichnet sich erfindungsgemäß dadurch aus, dass die Speichereinrichtung ein computerlesbares Programm aufweist, das den Prozessor dazu veranlasst, die nachfolgend erläuterten Schritte auszuführen, wenn das Programm auf dem Prozessor ausgeführt wird.

Zunächst wird mittels der Detektionsvorrichtung erfasst, ob in dem menschlichen oder tierischen Herz ein zu behandelndes kardiales Ereignis eingetreten ist. Bei diesem kardialen Ereignis kann es sich beispielsweise um eine Herzrhythmusstörung, wie etwa eine atriale oder ventrikuläre Tachykardie, oder einen Herzstillstand handeln.

Wenn ein zu behandelndes kardiales Ereignis detektiert wurde, erfolgt zunächst noch keine Behandlung dieses kardialen Ereignisses. Vielmehr wird zuvor eine Lage der Behandlungselektrode bestimmt. Statt einer Lage der Behandlungselektrode kann auch eine mit dieser Lage korrelierende Größe bestimmt werden. Als Lage einer Behandlungselektrode wird dabei die Lage eines leitenden Abschnitts der Behandlungselektrode verstanden. Ein solcher leitender Abschnitt wird auch als Elektrodenpol bezeichnet.

Anschließend wird die Lage der Behandlungselektrode oder die mit der Lage korrelierende Größe mit einer Referenzgröße verglichen. Eine kardiale Behandlung mittels der Behandlungseinrichtung und der Behandlungselektrode wird dann ausgeführt, wenn die Lage der Behandlungselektrode oder wenn die mit der Lage korrelierende Größe innerhalb einer vorgebbaren Toleranz mit der Referenzgröße übereinstimmt.

Stimmt die ermittelte Lage der Behandlungselektrode oder die mit der Lage korrelierende Größe nicht innerhalb einer vorgebbaren Toleranz mit der Referenzgröße überein, weist die Behandlungselektrode eine inadäquate Lage auf. Dann wird (zunächst) keine kardiale Behandlung ausgeführt. Denn dann könnte durch eine derartige kardiale Behandlung ein größerer Schaden als Nutzen erreicht werden. Wenn beispielsweise zwei Elektroden zu dicht beieinander liegen oder eine Elektrode nicht mehr im Atrium, sondern im Ventrikel eines Herzens angeordnet ist, könnten durch die Ausführung einer kardialen Behandlung unerwünschte, teilweise schwerwiegende Nebenwirkungen eintreten, die es zu vermeiden gilt.

Die Referenzgröße einschließlich ihrer Toleranz wird dabei derart bestimmt, dass eine Lage der Behandlungselektrode als inadäquat angesehen wird, wenn durch eine derartige Lage ein Behandlungserfolg unwahrscheinlich ist, eine Behandlungsabgabe Nebenwirkungen mit (gegebenenfalls nicht absehbaren) Folgen haben könnte oder wenn eine Behandlungsabgabe schädigende Folgen für das behandelte Herz haben könnte.

Die eigentlich erforderliche kardiale Behandlung wird jedenfalls so lange nicht ausgeführt, bis eine Entscheidung getroffen wurde, ob trotz der als inadäquat festgestellten Lage der Behandlungselektrode dennoch eine Behandlung durchgeführt werden soll. Denn mitunter kann selbst dann, wenn eine inadäquate Lage der Behandlungselektrode ermittelt wurde, der Nutzen einer dennoch ausgeführten kardialen Behandlung größer als ein zu erwartender Schaden sein. Daher ist in einer Variante die Möglichkeit eines manuellen oder automatisierten Eingriffs in das von dem Prozessor ausgeführte Programm vorgesehen, um dennoch eine kardiale Behandlung zu ermöglichen, auch wenn die festgestellte Lage der Behandlungselektrode zunächst gegen eine derartige Behandlung spricht. In Abwägung einer Schwere des detektierten zu behandelnden kardialen Ereignisses und der Abweichung der Lage der Behandlungselektrode von der Referenzgröße kann es jedoch zur Rettung des Lebens eines Patienten sinnvoll oder erforderlich sein, eine Behandlung auch bei einem fraglichen Therapieerfolg oder befürchteten (schwerwiegenden) Nebenwirkungen durchzuführen.

Die Anordnung eignet sich zur temporären oder dauerhaften Implantation, wobei insbesondere eine dauerhafte Implantation der Anordnung in einem menschlichen oder tierischen Patienten vorgesehen ist. Wenn die Anordnung einem tierischen Patienten implantiert werden soll, handelt es sich bei dem Patienten insbesondere um ein Säugetier, beispielsweise um ein Säugetier, das ausgewählt ist aus der Gruppe bestehend aus Nagetieren, Pferden, Hunden und Katzen.

Bei der Behandlungseinrichtung und der Behandlungselektrode, die auch als Therapieeinrichtung und Therapieelektrode bezeichnet werden können, kann es sich um eine Einrichtung und eine Elektrode handeln, die für jede beliebige Art der Behandlung eines menschlichen oder tierischen Herzens geeignet sind. Beispielsweise kann die Behandlungselektrode eine Stimulationselektrode sein, mit der menschliches oder tierisches Herzgewebe stimuliert werden kann. Die Behandlungselektrode kann auch eine Defibrillationselektrode sein. Die genaue Ausgestaltung der Behandlungselektrode ist für die vorliegend beanspruchte Erfindung nicht von besonderer Bedeutung.

In einer Variante weist die implantierbare Anordnung mehr als eine Behandlungseinrichtung und mehr als eine Behandlungselektrode auf. Insbesondere ist es vorgesehen, dass für jede zu behandelnde Herzregion eine Behandlungselektrode vorgesehen ist. Dabei können mehrere Behandlungselektroden mit einer einzigen Behandlungseinrichtung verbunden sein. Typischerweise ist jeder Behandlungselektrode aber genau eine Behandlungseinrichtung zugeordnet.

In einer alternativen Ausgestaltung weist die implantierbare Anordnung mehr als eine Detektionseinrichtung auf. Auch hier kann pro Herzregion, die behandelt werden soll, eine Detektionseinrichtung vorgesehen sein. Bei den unterschiedlichen zu behandelnden Herzregionen kann es sich beispielsweise um das linke Atrium, den linken Ventrikel, das rechte Atrium oder den rechten Ventrikel eines menschlichen oder tierischen Herzens handeln.

In einer Variante handelt es sich bei der zu bestimmenden Lage der Behandlungselektrode um eine relative Lage der Behandlungselektrode zu einem Referenzpunkt der Anordnung. Dieser Referenzpunkt kann beispielsweise eine weitere Behandlungselektrode der Anordnung sein. Es kann sich bei dem Referenzpunkt auch um eine Referenzelektrode der Anordnung handeln. Beispielsweise kann ein Teil des Gehäuses der implantierbaren Anordnung in Form einer Referenzelektrode ausgestaltet sein.

In einer Variante wird die relative Lage der Behandlungselektrode bzw. die mit dieser relativen Lage korrelierende Größe dadurch bestimmt, dass ein Abstand zwischen der Behandlungselektrode und dem Referenzpunkt bestimmt wird. Dieser Abstand wird dann mit einem Referenzabstand, der als Referenzgröße dient, verglichen. Durch eine derartige Abstandsbestimmung lässt sich ermitteln, ob eine relative Verschiebung der betrachteten Elektroden stattgefunden hat. Es ist dabei möglich, einen einzigen Abstand zwischen der Behandlungselektrode und dem Referenzpunkt oder aber zwei oder mehr Abstände zwischen unterschiedlichen Behandlungselektroden und/oder zwischen einer oder mehrerer Behandlungselektrode und einer Referenzelektrode zu bestimmen. Dabei ist es möglich, die einzelnen Abstände bei der Berechnung einer mit dem Abstand korrelierenden Größe unterschiedlich stark zu gewichten. Auf diese Weise lässt sich eine relative Lageänderung einer Elektrode, bei der eine Lageänderung besonders schwerwiegende Folgen für einen Behandlungserfolg haben kann, stärker gewichten als eine relative Lageänderung einer Elektrode, bei der eine Lageänderung einen weniger schwerwiegenden Effekt auf einen Behandlungserfolg haben kann.

Es gibt grundsätzlich unterschiedliche Möglichkeiten, die absolute oder relative Lage der Behandlungselektrode zu ermitteln. In einer Variante veranlasst das Programm den Prozessor dazu, die relative Lage der Behandlungselektrode durch eine Messung eines elektrischen Stroms und/oder durch eine Messung einer elektrischen Spannung zwischen der Behandlungselektrode und dem Referenzpunkt zu bestimmen. Wenn beispielsweise der Abstand zwischen unterschiedlichen Behandlungselektroden oder zwischen einer Behandlungselektrode und einer Referenzelektrode bestimmt werden soll, wird eine Spannung zwischen diesen Elektroden angelegt. Eine der Elektroden dient dabei als Stromsenke. Es werden dann jeweils Elektrodenpaare aus zwei der betrachteten n Elektroden ausgewählt und die zwischen diesen Elektroden anliegende Spannung bestimmt. Wenn die implantierbare Anordnung insgesamt n Elektroden (Behandlungselektroden und Referenzelektroden) aufweist, ergeben sich somit $\frac{n!}{(n-2)!2!} = \binom{n}{2}$ Elektrodenkombinationen, die für eine entsprechende Spannungsbestimmung herangezogen werden können. Gibt es beispielsweise n=4 Elektroden, so ergeben sich

$$\binom{4}{2} = \frac{4*3*2*1}{2*1*2*1} = 6$$

Kombinationen für eine Auswahl von zwei Elektroden.

In einer Ausführungsform der Erfindung ist es vorgesehen, zwischen mindesten zwei bis maximal n-1 Elektrodenpolen elektrische Spannungen anzulegen. Anschließend wird eine Messung der Spannung wird zwischen Elektrodenpolen durchgeführt, von denen mindestens ein Pol nicht an der Spannungserzeugung beteiligt ist.

Des Weiteren, gemäß einer weiteren Ausführungsform der Erfindung, wird zwischen mindesten zwei bis maximal n-1 Elektrodenpolen ein elektrischer Strom eingespeist. Mindestens ein Elektrodenpol dient dabei als Stromsenke. Anschließend wird eine Messung der Spannung oder des Stroms wird zwischen mindestens zwei der n Elektrodenpole durchgeführt.

Gemäß einem bevorzugten Ausführungsbeispiel der Erfindung wird der elektrische Strom und/oder die Spannung über mindestens eine Behandlungselektrode (340, 341, 342, 640) abgegeben und mittels mindestens einer Behandlungselektrode (340, 341, 342, 640) und/oder Referenzelektrode gemessen. Die Messung erfolgt unmittelbar nach der Abgabe des elektrischen Stroms und/oder der Spannung, damit ein durch den eigespeisten Strom / die angelegte Spannung erzeugter Effekt direkt gemessen werden kann. Beim Effekt kann es sich um eine durch den Strom/die Spannung erzeugte physiologische Wirkung handeln.

Der Referenzpunkt bzw. die Referenzelektrode befindet sich in einer Variante im Bereich des Thorax des Patienten. Wie bereits ausgeführt, kann ein Teil des Gehäuses der implantierbaren Anordnung als Referenzelektrode dienen. Dann ist der Abstand zwischen den im Herzen angeordneten Elektroden und der Referenzelektrode verhältnismäßig groß.

Die an die Elektroden angelegte Spannung weist in einer Variante eine Frequenz in einem Bereich von 0 bis 1 MHz, insbesondere 0,1 bis 0,9 MHz, insbesondere 0,2 bis 0,8 MHz, insbesondere 0,3 bis 0,7 MHz, insbesondere 0,4 bis 0,6 MHz, insbesondere 0,5 bis 1 MHz auf.

In einer Variante handelt es sich bei der an die Elektroden angelegten Spannung um eine gepulste Spannung mit einer Pulsdauer in einem Bereich von 10 $\mu$s bis 100 ms, insbesondere 100 $\mu$s bis 10 ms, insbesondere 500 $\mu$s bis 5 ms, insbesondere 750 $\mu$s bis 2,5 ms, insbesondere 900 $\mu$s bis 2 ms, insbesondere 1 ms bis 1,5 ms.

In einer Variante können die Abstände zwischen den einzelnen Pulsen einen sehr breiten zeitlichen Bereich abdecken. Beispielsweise ist es möglich, in sehr kurzer Folge hintereinander eine Vielzahl von Pulsen zur Bestimmung der Lage der Behandlungselektrode abzugeben, um anschließend bis zur nächsten Lagebestimmung eine längere Pause einzulegen. Somit können die Zeiten, die zwischen einzelnen Pulsen vergehen, in einer Variante 10 $\mu$s bis 2 Jahre, insbesondere 50 $\mu$s bis 1,9 Jahre, insbesondere 100 $\mu$s bis 1,8 Jahre, insbesondere 1 ms bis 1,7 Jahre, insbesondere 500 ms bis 1,6 Jahre, insbesondere 1 Sekunde bis 1,5 Jahre, insbesondere 10 Sekunden bis 1,4 Jahre, insbesondere 1 Minute bis 1,3 Jahre, insbesondere 10 Minuten bis 1,2 Jahre, insbesondere 1 Stunde bis 1 Jahr, insbesondere 1 Tag bis 0,5 Jahre, insbesondere 1 Woche bis 0,25 Jahre, insbesondere 2 Wochen bis 1 Monat betragen. Dabei ist es auch denkbar, dass die Pulsabstände während einzelnen Messungen in einem aus den kürzeren Zeitabständen bestehenden Intervall (beispielsweise 10 $\mu$s bis 1 Sekunde) und die Abstände zwischen Pulsen unterschiedlicher Messungen in einem Intervall aus den längeren angegebenen Zeitabschnitten (beispielsweise 1 Minute bis 2 Jahre) liegen. Selbstverständlich können sämtliche der vorstehend explizit erwähnten Intervalle oder die sich aus den oberen und unteren Werten, die diese Intervalle begrenzen, anderweitig zu bildendenden Intervalle für die Dauer der Pulsabstände herangezogen werden.

In einer Ausgestaltung weist eine Messung eine Vielzahl einzelner Messpulse auf, die in Form eines sogenannten Bursts abgegeben werden.

In einer Variante erfolgt mindestens eine Messung pro Jahr, um eine kontinuierliche Lageüberwachung der Behandlungselektrode zu ermöglichen.

In einer Variante erfolgt die Messung der an die Elektroden angelegten Spannung bzw. der in die Elektroden eingeleiteten Ströme in Form einer Impedanzmessung. Hierzu können beispielsweise 2-Pol-, 3-Pol- und/oder 4-Pol-Impedanzmessverfahren angewendet werden.

Die Messung des in die Elektroden eingespeisten Stroms bzw. der an die Elektroden angelegten Spannung kann in einer Variante direkt über die im Körper des Patienten erzeugten elektrischen Felder erfolgen. Darüber hinaus ist in einer alternativen Ausgestaltung auch eine indirekte Messung angedacht. Denn durch die angelegte Spannung bzw. den eingespeisten Strom wird eine Erregung einer Herzregion, an der eine der an der Messung beteiligte Elektroden positioniert ist, erzeugt, die sich nachfolgend zu einer anderen Herzregion ausbreitet. Dabei kommt es zu einer physiologischen Verzögerung der Erregungsausbreitung. Anhand des Musters der Erregungsausbreitung und/oder der physiologischen Verzögerung der Erregungsausbreitung lässt sich die Wirkung des eingespeisten Stroms bzw. der angelegten Spannung bestimmen.

Der Strom bzw. die Spannung erzeugten elektrische Felder, die eine bestimmte Wirkung im Körper hervorrufen. Diese Wirkung und/oder die indirekt über die verzögerte physiologische Wirkung des Stroms bzw. der Spannung bestimmte Wirkung stellt einen für die Lage der Behandlungselektrode, insbesondere für den Abstand der Behandlungselektrode zu einem Referenzpunkt, wie einer Referenzelektrode, charakteristischen Parameter dar. Dieser Parameter ist eine mit der Lage korrelierende Größe im Sinne der vorliegenden Erfindung.

In einer alternativen Ausgestaltung ist es auch angedacht, sowohl eine sofortige (unmittelbare) Wirkung des eingespeisten Stroms bzw. der angelegten Spannung als auch eine verzögerte physiologische Wirkung des Stroms bzw. der Spannung messtechnisch zu erfassen und zur Bestimmung der Lage der Behandlungselektrode heranzuziehen.

In einer Alternative wird der Prozessor durch das Programm dazu veranlasst, die erfasste verzögerte physiologische Wirkung zu bewerten. So ist insbesondere vorgesehen, einen Plausibilitätscheck durchzuführen, um zu überprüfen, ob die detektierte physiologische Verzögerung durch die angenommene Lage der Behandlungselektrode zu begründen ist. Für diesen Plausibilitätscheck können Grenzwerte vorgegeben werden, die innerhalb eines ebenfalls vorgebbaren Toleranzbereichs eingehalten werden müssen. Falls eine viel zu große verzögerte physiologische Wirkung oder eine viel zu kleine verzögerte physiologische Wirkung detektiert wird, kann dieser Plausibilitätscheck dazu führen, dass das gewonnene Ergebnis noch nicht von vornherein als außerhalb des einzuhaltenden Bereichs angesehen wird. Vielmehr kann dies ein Hinweis darauf sein, dass die Messung fehlerbehaftet ist und erneut durchgeführt werden muss. Demgegenüber spricht insbesondere dann, wenn im Rahmen des Plausibilitätschecks nur geringe Abweichungen von der vermuteten Lage festgestellt werden, vieles dafür, dass die messtechnisch ermittelte Lage der Behandlungselektrode ihrer tatsächlichen Lage entspricht. Ob diese Lage dann auch tatsächlich innerhalb der vorgebbaren Toleranz mit der Referenzgröße für diese Lage übereinstimmt, kann in einem nachfolgenden Schritt ermittelt werden.

Wann immer im Rahmen der vorliegenden Anmeldung ein vorgebbarer Wert beschrieben oder erläutert wird, kann dieser Wert beispielsweise fest vorgegeben oder frei programmierbar sein. Es ist in einem solchen Fall auch möglich, dass ein entsprechender Wert aus einer Vielzahl von beispielhaften Werten ausgewählt werden kann. Diese beispielhaften Werte können in für die jeweilige Anwendung sinnvollen Bereichen vordefiniert sein und beispielsweise in erfahrungsgemäß praxisnahen Intervallen zur Auswahl bereitgestellt werden.

In einer Variante wird anhand einer unmittelbaren Wirkung der im Körper erzeugten elektrischen Felder ermittelt, ob ein erlaubter Mindestabstand zwischen zwei Elektroden eingehalten oder unterschritten wird. Beispielsweise kann geprüft werden, ob ein infolge einer angelegten Spannung bzw. eines eingespeisten Stroms ermitteltes Messsignal einen als Referenzgröße dienenden Schwellenwert nicht überschreitet. Wird der Schwellenwert nicht überschritten, ist der Mindestabstand eingehalten.

In einer Variante kann die Sensitivität der Ermittlung eines Abstandes zwischen zwei Elektroden dadurch erhöht werden, dass verschiedene Messverfahren miteinander kombiniert werden. Beispielsweise kann dann, wenn bereits durch Ermittlung der sofortigen Wirkung der angelegten Spannung bzw. des eingespeisten Stroms das Ergebnis erhalten wurde, dass ein Mindestabstand nicht unterschritten wurde, zusätzlich eine Prüfung erfolgen, ob die durch die angelegte Spannung bzw. den eingespeisten Strom erreichte physiologisch verzögerte Wirkung eine vorgebbare Verzögerungszeit nicht unterschreitet. Wenn auch die definierte Verzögerungszeit nicht unterschritten wird, liegen zwei mit unterschiedlichen Messverfahren erhaltene Bestätigungen darüber vor, dass ein Mindestabstand zwischen den Elektroden nicht unterschritten ist.

In einer Variante ist es vorgesehen, dass es sich bei der Lage der Behandlungselektrode um eine absolute Lage der Behandlungselektrode innerhalb eines menschlichen oder tierischen Herzens handelt, wenn die Anordnung in einem Menschen oder einem Tier implantiert ist. Das heißt, dass die Lage in diesem Fall unabhängig von anderen Bauteilen der dann implantierten Anordnung bestimmt wird. Dabei bezeichnet der Begriff "absolute Lage" die Anordnung der Behandlungselektrode im Herzen bzw. in einer bestimmten Herzregion.

Um eine derartige absolute Lagebestimmung der Behandlungselektrode zu ermöglichen, weist die Behandlungselektrode in einer Variante einen Beschleunigungssensor auf. Mit einem derartigen Beschleunigungssensor, der beispielsweise als linearer Beschleunigungssensor oder als Drehratensensor bzw. Gyroskop ausgestaltet sein kann, kann eine Bewegung der Behandlungselektrode innerhalb des Herzens nachverfolgt werden.

In einer Variante ist es hierfür vorgesehen, dass das Programm den Prozessor dazu veranlasst, mittels Beschleunigungsdaten, die durch den Beschleunigungssensor gewonnen wurden, ein Beschleunigungsprofil der Behandlungselektrode zu erstellen. Darüber hinaus wird der Prozessor dazu veranlasst, dieses Beschleunigungsprofil mit dem Herzrhythmus des menschlichen oder tierischen Herzens, in dem sich die entsprechende Behandlungselektrode befindet, zu korrelieren. Auf diese Weise ist es möglich, zu ermitteln, ob die Behandlungselektrode die Bewegungen der Herzregion, in der die Behandlungselektrode angeordnet ist bzw. vermutet wird, nachvollzieht. Ist das Beschleunigungsprofil einer ventrikulären Behandlungselektrode synchron zu dem ventrikulären Herzrhythmus des entsprechenden Herzventrikels, befindet sich die Behandlungselektrode nach wie vor in dem Ventrikel, in dem sie ursprünglich angeordnet wurde. Ergibt sich aus einer Korrelation des Beschleunigungsprofils der Behandlungselektrode und dem Herzrhythmus einer bestimmten Herzregion hingegen, dass die Behandlungselektrode die Bewegungen einer Herzregion nachvollzieht, in der sie gar nicht angeordnet sein sollte (beispielsweise vollzieht eine atriale Behandlungselektrode einen ventrikulären Herzrhythmus nach), zeigt dies, dass sich die Behandlungselektrode nicht mehr in der Herzregion befindet, in der sie ursprünglich angeordnet war. Das heißt, es hat eine Dislokation der Behandlungselektrode stattgefunden, die ein weiteres Ansprechen dieser Behandlungselektrode zur Durchführung einer kardialen Therapie regelmäßig ausschließt. Denn dann würde sich der erwartete Therapieerfolg nicht einstellen. Vielmehr wäre mit (schwerwiegenden) Nebenwirkungen einer entsprechenden kardialen Behandlung zu rechnen. Hier müsste zunächst eine Relokation der Behandlungselektrode durchgeführt werden, damit der erwünschte Behandlungserfolg auch tatsächlich erreicht werden kann.

In einer Variante werden bei der Bestimmung der absoluten Lage der Behandlungselektrode auch Daten von Sensoren erfasst, die außerhalb des menschlichen oder tierischen Herzens angeordnet sind und von den Bewegungen des Herzens nicht beeinflusst werden. Durch die Hinzuziehung derartiger ergänzender Sensordaten lassen sich anderweitig etwaig auftretende Artefakte reduzieren oder sogar eliminieren. Dies erhöht die Genauigkeit der Bestimmung der absoluten Lage der Behandlungselektrode.

In einer Variante weist die Anordnung einen Patientenzustandssensor auf. Ein solcher Patientenzustandssensor dient dazu, eine Körperlage oder einen Aktivitätszustand eines Patienten, dem die Anordnung implantiert wurde, zu bestimmen. Ein solcher Patientenzustandssensor kann beispielsweise ein Lagesensor sein, mit dem ermittelt werden kann, ob sich der Patient in einer aufrechten Lage oder einer waagerechten Lage befindet. Ein zur Bestimmung des Aktivitätszustands des Patienten geeigneter Patientenzustandssensor ist beispielsweise ein Sensor, mit dem die Herzfrequenz des Patienten überwacht werden kann. Die Herzfrequenz wird typischerweise direkt durch eine oder mehrere Elektroden der Anordnung zur Behandlung des Herzens überwacht, sodass die bereits in der Anordnung vorhandene Sensorik die Funktion des Patientenzustandssensors übernehmen kann, weshalb in einem solchen Fall ein gesonderter Patientenzustandssensor nicht unbedingt erforderlich ist. Mittels der Erfassung der Körperlage oder des Aktivitätszustand des Patienten lassen sich zusätzliche Daten gewinnen, die die Verlässlichkeit der übrigen Messdaten erhöhen, da sie eine Erklärung für möglicherweise ungewöhnliche Messdaten liefern können.

In einer Variante veranlasst das Programm den Prozessor dazu, einen Verlauf der Lage der Behandlungselektrode über die Zeit zu speichern. Auf diese Weise ist es möglich, einen Trend der Lage der Behandlungselektrode festzustellen. Beispielsweise lässt sich so bereits frühzeitig eine beginnende Dislokation der Behandlungselektrode erkennen, wenn eine an sich noch innerhalb des vorgegebenen Toleranzbereichs erfolgende Veränderung der Lage der Behandlungselektrode festgestellt wird und gleichzeitig erkennbar ist, dass diese Lagerveränderung in genau eine Richtung (beispielsweise ein sich vergrößernder Abstand oder ein sich verkleinernder Abstand) erfolgt. Durch eine derartige Verlaufsanalyse der Lage der Behandlungselektrode kann auch die Lagestabilität der Behandlungselektrode ermittelt werden. Auf diese Weise lässt sich beispielsweise ein drohender Wackelkontakt der Behandlungselektrode frühzeitig erkennen.

In einer Variante veranlasst das Programm den Prozessor dazu, nur solche Werte der Lage der Behandlungselektrode zu speichern, die gewonnen wurden, wenn sich der Patient in einer definierten Körperlage und/oder in einem definierten Aktivitätszustand befunden hat. Zu diesem Zwecke werden vorzugsweise die Daten, die von dem optional vorgesehenen Patientenzustandssensor gewonnen wurden, herangezogen. Durch eine Einschränkung der zu speichernden Werte der Lage der Behandlungselektrode kann ein insgesamt verlässlicherer Trend einer Veränderung der Lage der Behandlungselektrode ermittelt werden. Denn wenn die Messungen, die zur Beurteilung der Lage der Behandlungselektrode gewonnen wurden, nur dann gespeichert werden, wenn sie sich auf eine vergleichbare Körperlage des Patienten bzw. einen vergleichbaren Aktivitätszustand des Patienten beziehen, wird eine insgesamt bessere Vergleichbarkeit der gespeicherten Werte erreicht. Dann können genauere Aussagen über die Lagestabilität der Behandlungselektrode getroffen werden. Artefakte werden auf diese Art und Weise minimiert oder eliminiert.

In einer Variante veranlasst das Programm den Prozessor dazu, eine Lagebestimmung der Behandlungselektrode auch dann durchzuführen, wenn keine kardiale Behandlung des entsprechenden menschlichen oder tierischen Herzens durchgeführt werden soll. Auf diese Weise lassen sich auch unabhängig von einer kardialen Behandlung Messdaten gewinnen, die einen Aufschluss über die Lage der Behandlungselektrode geben. Werden derartige Daten in regelmäßigen oder unregelmäßigen Intervallen gewonnen, können Aussagen zur Lagestabilität der Behandlungselektrode getroffen werden. So lässt sich frühzeitig eine (beginnende) Dislokation der Behandlungselektrode erkennen, die dann gegebenenfalls noch vor der Notwendigkeit einer kardialen Behandlung korrigiert werden kann.

In einer Variante veranlasst das Programm den Prozessor dazu, nach Ablauf einer voreinstellbaren Zeit oder in Reaktion auf ein Signal eine kardiale Behandlung durchzuführen, wenn zuvor ein zu behandelndes kardiales Ereignis festgestellt wurde. Dabei erfolgt diese kardiale Behandlung in einem solchen Fall mittels der Behandlungseinrichtung und der Behandlungselektrode unabhängig von der zuvor ermittelten Lage der Behandlungselektrode. Das heißt, auch dann, wenn eine inadäquate Lage der Behandlungselektrode ermittelt wurde, ermöglicht dieser Sicherheitsschritt dennoch eine kardiale Behandlung. Denn mitunter ist die festgestellte Abweichung der Lage der Behandlungselektrode von der erwarteten Lage verhältnismäßig gering, das zu behandelnde kardiale Ereignis gleichzeitig aber verhältnismäßig schwerwiegend. Dann kann in Abwägung des Patienteninteresses die Durchführung einer kardialen Behandlung unter Inkaufnahme von Nebenwirkungen insgesamt vorteilhafter sein als die Unterbindung einer derartigen kardialen Behandlung.

Ein Signal, das den Prozessor dazu veranlasst, eine kardiale Behandlung unabhängig von der ermittelten Lage der Behandlungselektrode durchzuführen, kann beispielsweise ein externes Eingabesignal sein, das von einem Arzt erzeugt wird. Somit hat ein Arzt - unter Abwägung des Patienteninteresses und des medizinischen Risikos einer kardialen Behandlung - die Möglichkeit, trotz einer anscheinend oder tatsächlich dislozierten Behandlungselektrode dennoch eine kardiale Behandlung durchzuführen.

In einer Variante weist die Anordnung mindestens eine Vorrichtung zum Aussenden und/oder Empfangen akustischer Wellen auf. Dabei wird diese Vorrichtung zur Bestimmung der Lage der Behandlungselektrode eingesetzt. Bei den akustischen Wellen kann es sich beispielsweise um Ultraschallwellen handeln. Beispielsweise kann ein Emitter für akustische Wellen an der Behandlungselektrode angeordnet sein. Ein Empfänger für die von dem Emitter ausgesandten akustischen Wellen kann am Referenzpunkt angeordnet sein. Über eine nach der Aussendung der akustischen Wellen erfolgende Signaldämpfung und/oder eine Phasenverschiebung im Vergleich zum eingespeisten akustischen Signal können dann zusätzliche Informationen zum Abstand zwischen der Behandlungselektrode und dem Referenzpunkt gewonnen werden. Die Verzögerung, die sich infolge der akustischen Signalausbreitung im Herzen bzw. im Körper des Patienten ergibt, ist um Größenordnungen geringer als eine physiologische Verzögerung der Erregungsausbreitung infolge des Einbringens eines Strompulses in das Herz. Somit kann über eine kombinierte Auswertung der sofortigen Wirkung eines eingespeisten Stroms bzw. einer angelegten Spannung mittels der dadurch erzeugten elektrischen Felder im Herz und/oder der verzögerten physiologischen Erregung infolge der angelegten Spannung bzw. des eingebrachten Stroms und/oder der ausgesendeten und empfangenen akustischen Signale in bis zu drei voneinander verschiedenen Zeitebenen gearbeitet werden, was eine sehr genaue Lagebestimmung der Behandlungselektrode ermöglicht.

Es ist auch möglich, den Emitter der akustischen Wellen bzw. akustischen Signale nicht an der Elektrode, sondern am Referenzpunkt anzuordnen und den Empfänger an der Elektrode. Gleichfalls ist es möglich, statt eines von dem Emitter getrennten Empfängers einen Transceiver für akustische Signale einzusetzen. Es ist auch möglich, den Emitter, den Empfänger oder den Transceiver nicht an der Elektrode, sondern an einem anderen Bauteil der implantierbaren Anordnung anzubringen. In einem solchen Fall ist es sinnvoll, an der Elektrode ein reflektierendes Element vorzusehen, das die auf die Elektrode gerichteten akustischen Signale reflektiert. Aus dem Verlauf der akustischen Signale zur Elektrode hin und/oder von der Elektrode weg können auch in einem solchen Fall relevante Daten für die Bestimmung des Abstandes der Elektrode zu dem Emitter, dem Empfänger bzw. dem Transceiver zu gewinnen.

In einer Variante weist die Anordnung mindestens eine Vorrichtung zum Aussenden und/oder Empfangen elektromagnetischer Wellen auf. Diese Ausführungsform der Vorrichtung dient der Bestimmung der Lage der Behandlungselektrode Abstandsmessung mit elektromagnetischen Wellen. Die elektromagnetischen Wellen können dabei eine Frequenz im Bereich von 1 MHz bis 1 GHz, insbesondere von 10 MHz bis 900 GHz, insbesondere von 100 MHz bis 800 GHz, insbesondere von 500 MHz bis 700 GHz, insbesondere von 1 GHz bis 600 GHz, insbesondere von 10 GHz bis 500 GHz, insbesondere von 100 GHz bis 400 GHz, insbesondere von 200 GHz bis 300 GHz aufweisen. Die elektromagnetischen Wellen werden galvanisch in das Gewebe eingespeist.

In einer Variante werden die elektromagnetischen Wellen im Frequenzbereich von 300 MHz bis zum Infrarotbereich, d.h. 300 GHz in das Herzgewebe eingespeist. Anschließend kann auf Grundlage einer Transmissionsmessung der elektromagnetischen Wellen ein Abstand zwischen der Behandlungselektrode und dem Referenzpunkt ermittelt werden. Gemäß einem Ausführungsbeispiel der vorliegenden Erfindung können anstatt akustischer Wellen elektromagnetische Wellen im sichtbaren Frequenzbereich verwendet werden. Geeignete Frequenzbereiche liegen zwischen 400 THz und 800 THz, insbesondere zwischen 500 THz und 700 THz, insbesondere zwischen 550 THz und 650 THz. Auch im Fall von elektromagnetischen Wellen können Emitter und Empfänger oder Transceiver eingesetzt werden. Eine Anordnung eines Emitters, eines Empfängers oder eines Transceivers an der Elektrode ist möglich, aber nicht zwingend. Der Einsatz von reflektierenden Elementen, die die elektromagnetischen Wellen reflektieren, ist ebenfalls möglich, aber nur für bestimmte Frequenzbereiche sinnvoll einsetzbar. Denn insbesondere Licht im sichtbaren Bereich durchdringt nur kurze Strecken menschlichen Gewebes, sodass die Lichtintensität beim Erreichen eines reflektierenden Elementes möglicherweise schon zu gering ist. Elektromagnetische Wellen niedrigerer Frequenz weisen in menschlichem Gewebe jetzt jedoch eine größere Reichweite auf, sodass hier Reflexionen erfolgen können. Statt einfacher Transmissionsmessungen sind auch kombinierte Reflexions- und Transmissionsmessungen möglich.

In einer Variante handelt es sich bei dem zu erfassenden kardialen Ereignis, das mittels der implantierbaren Anordnung behandelt werden soll, um eine atriale Tachykardie. In einem solchen Fall handelt es sich bei der kardialen Behandlung um eine atriale anti-tachykarde Stimulation. Die Behandlungselektrode kann in einem solchen Fall auch als Stimulationselektrode bezeichnet werden.

Ein Aspekt der vorliegenden Erfindung betrifft ein Verfahren zur Steuerung des Betriebs einer implantierbaren Anordnung zur Behandlung eines menschlichen oder tierischen Herzens, insbesondere einer implantierbaren Anordnung gemäß den vorstehenden Erläuterungen. Dieses Verfahren weist die nachfolgend erläuterten Schritte auf.

Zunächst wird mittels einer Detektionseinrichtung erfasst, ob in einem menschlichen oder tierischen Herz ein zu behandelndes kardiales Ereignis eingetreten ist.

Wenn ein zu behandelndes kardiales Ereignis eingetreten ist, wird eine Lage einer Behandlungselektrode bestimmt. Alternativ oder zusätzlich kann eine mit dieser Lage der Behandlungselektrode korrelierende Größe bestimmt werden.

Nachfolgend erfolgt ein Vergleich der Lage der Behandlungselektrode oder der mit der Lage korrelierenden Größe mit einer Referenzgröße.

Wenn die Lage der Behandlungselektrode oder die mit der Lage korrelierende Größe innerhalb einer vorgebbaren Toleranz mit der Referenzgröße übereinstimmt, wird in einer Behandlungseinrichtung der Anordnung ein Impuls für eine kardiale Behandlung erzeugt.

Dieser Impuls kann dann über eine Behandlungselektrode weitergeleitet werden.

Stimmt die Lage der Behandlungselektrode oder die mit der Lage korrelierende Größe der Behandlungselektrode hingegen nicht innerhalb einer vorgebbaren Toleranz mit der Referenzgröße überein, wird in der Behandlungseinrichtung kein Impuls für eine nachfolgende kardiale Behandlung erzeugt.

Ein Aspekt der vorliegenden Erfindung betrifft ein nicht-flüchtiges Computerprogrammprodukt mit einem computerlesbaren Code, der einen Prozessor dazu veranlasst, die nachfolgend erläuterten Schritte auszuführen, wenn er auf dem Prozessor ausgeführt wird.

Zunächst wird mittels einer Detektionsvorrichtung erfasst, ob in dem menschlichen oder tierischen Herz ein zu behandelndes kardiales Ereignis eingetreten ist.

Wenn ein zu behandelndes kardiales Ereignis detektiert wurde, erfolgt zunächst noch keine Behandlung dieses kardialen Ereignisses. Vielmehr wird zuvor eine Lage einer Behandlungselektrode bestimmt. Statt einer Lage der Behandlungselektrode kann auch eine mit dieser Lage korrelierende Größe bestimmt werden.

Anschließend wird die Lage der Behandlungselektrode oder die mit der Lage korrelierende Größe mit einer Referenzgröße verglichen. Eine kardiale Behandlung mittels einer Behandlungseinrichtung, der die Behandlungselektrode zugeordnet ist, wird dann ausgeführt, wenn die Lage der Behandlungselektrode oder wenn die mit der Lage korrelierende Größe innerhalb einer vorgebbaren Toleranz mit der Referenzgröße übereinstimmt.

Stimmt die ermittelte Lage der Behandlungselektrode oder die mit der Lage korrelierende Größe nicht innerhalb einer vorgebbaren Toleranz mit der Referenzgröße überein, weist die Behandlungselektrode eine inadäquate Lage auf. Dann wird (zunächst) keine kardiale Behandlung ausgeführt. Denn dann könnte durch eine derartige kardiale Behandlung ein grö-ßerer Schaden als Nutzen erreicht werden.

Ein weiterer Aspekt, der jedoch nicht beansprucht wird, betrifft ein Verfahren zur Behandlung eines menschlichen oder tierischen Patienten, der eine solche Behandlung benötigt, mittels einer implantierbaren Anordnung zur Behandlung eines menschlichen oder tierischen Herzens, wobei die Anordnung einen Prozessor, eine Speichereinrichtung, eine Behandlungseinrichtung mit einer Behandlungselektrode und eine Detektionseinrichtung zum Detektieren eines behandlungsbedürftigen kardialen Ereignisses aufweist. Das Verfahren umfasst die nachfolgend erläuterten Schritte.

Zunächst wird mittels der Detektionseinrichtung erfasst, ob in dem Herz des Patienten ein zu behandelndes kardiales Ereignis eingetreten ist.

Wenn ein zu behandelndes kardiales Ereignis eingetreten ist, wird eine Lage der Behandlungselektrode oder eine mit dieser Lage korrelierende Größe bestimmt.

Dann wird die Lage der Behandlungselektrode oder der mit der Lage korrelierenden Größe mit einer Referenzgröße verglichen.

wenn die Lage der Behandlungselektrode oder die mit der Lage korrelierende Größe innerhalb einer vorgebbaren Toleranz mit der Referenzgröße übereinstimmt, wird mittels der Behandlungseinrichtung und der Behandlungselektrode eine kardiale Behandlung ausgeführt.

Wenn die Lage der Behandlungselektrode oder die mit der Lage korrelierende Größe hingegen nicht innerhalb einer vorgebbaren Toleranz mit der Referenzgröße übereinstimmt, wird mittels der Behandlungseinrichtung und der Behandlungselektrode jedenfalls zunächst keine kardiale Behandlung ausgeführt.

Ein Aspekt der vorliegenden Erfindung betrifft eine implantierbare Anordnung zur diagnostischen und/oder therapeutischen Behandlung eines menschlichen oder tierischen Patienten mit den nachfolgend erläuterten Merkmalen. Eine solche Anordnung weist einen Prozessor, eine Speichereinrichtung, eine Behandlungseinrichtung und eine Datenfernübertragungseinrichtung auf. Die Behandlungseinrichtung dient dazu, eine diagnostische und/oder therapeutische Behandlung des Patienten, dem die Anordnung implantiert wurde, vorzunehmen. Mittels der Datenfernübertragungseinrichtung können über an sich bekannte Datenfernübertragungsnetzwerke wie etwa WLAN oder Mobilfunk insbesondere drahtlos Daten zu einem sich von der implantierbaren Anordnung entfernt befindenden Empfänger gesendet werden.

Erfindungsgemäß ist vorgesehen, dass die Speichereinrichtung ein computerlesbares Programm aufweist, das den Prozessor dazu veranlasst, die nachfolgend erläuterten Schritte auszuführen, wenn das Programm auf dem Prozessor ausgeführt wird.

Zunächst wird ermittelt, ob durch eine Behandlungsfunktionalität der Behandlungseinrichtung eine Gefährdung eines Patienten, dem die Anordnung implantiert wurde, dann auftreten könnte, wenn mittels der Behandlungseinrichtung eine diagnostische und/oder therapeutische Behandlung des Patienten durchgeführt würde, die der Behandlungsfunktionalität entspricht. Es wird also vorab ermittelt, ob eine solche Patientengefährdung bei einer geplanten (aber noch nicht durchgeführten) Behandlung vorliegen könnte.

Wenn eine derartige mögliche Patientengefährdung ermittelt wurde, wird die entsprechende Behandlungsfunktionalität deaktiviert. Die Behandlungseinrichtung weist typischerweise eine Vielzahl unterschiedlicher Behandlungsfunktionalitäten auf. Jede einzelne dieser Behandlungsfunktionalitäten lässt sich typischerweise unabhängig von anderen Behandlungsfunktionalitäten deaktivieren. Es ist auch möglich, dass bestimmte Behandlungsfunktionalitäten eine Gruppe bilden, die gemeinsam deaktiviert wird.

Die Deaktivierung hat zur Folge, dass eine durch die Behandlungsfunktionalität definierte therapeutische oder diagnostische Behandlung des Patienten nicht mehr durch die Behandlungseinrichtung durchgeführt werden kann. Dies ist erst dann wieder möglich, wenn die Behandlungsfunktionalität reaktiviert wurde. Wie bereits eingangs ausgeführt, ist es bei den aus dem Stand der Technik bekannten Lösungen erforderlich, dass ein Patient einen Spezialarzt aufsucht, um eine derartige Reaktivierung der Behandlungsfunktionalität vornehmen zu lassen.

Gemäß der vorliegend beanspruchten Erfindung erfolgt nun jedoch ein Schritt des Empfangens von Reaktivierungsdaten mittels der Datenfernübertragungseinrichtung. Es ist mit der vorliegend beanspruchten Erfindung also möglich, der implantierbaren Anordnung über die Datenfernübertragungseinrichtung Reaktivierungsdaten zu senden.

Dann erfolgt eine Reaktivierung der deaktivierten Behandlungsfunktionalität auf der Grundlage der empfangenen Reaktivierungsdaten. Wenn zuvor verschiedene Behandlungsfunktionalitäten deaktiviert wurden, können die Reaktivierungsdaten Informationen darüber enthalten, welche der deaktivierten Behandlungsfunktionalitäten reaktiviert werden sollen. Es ist auch möglich, dass stets alle zuvor deaktivierten Behandlungsfunktionalitäten durch die empfangenen Reaktivierungsdaten reaktiviert werden.

Eine Reaktivierung der Behandlungsfunktionalität hat zur Folge, dass eine der Behandlungsfunktionalität entsprechende diagnostische und/oder therapeutische Behandlung anschließend wieder durchgeführt werden kann.

Es ist dabei vorgesehen, dass die Reaktivierungsdaten nur von medizinischem, insbesondere ärztlichem, Fachpersonal, das auf derartige implantierbare Anordnungen spezialisiert ist, an die implantierbare Anordnung gesendet werden können. Dann kann sichergestellt werden, dass eine Reaktivierung der Behandlungsfunktionalität nur dann erfolgt, wenn nicht mehr von einer Patientengefährdung ausgegangen werden kann. Gleichzeitig ist es jedoch nicht erforderlich, dass der Patient beim entsprechenden Arzt oder medizinischen Fachpersonal vorstellig wird. Somit werden für den Patienten unnötige Wege und für den Arzt nicht erforderliche Patientenbesuche vermieden.

In einer Variante veranlasst das Programm den Prozessor dazu, mittels der Datenfernübertragungseinrichtung Zustandsdaten zu senden. Diese Zustandsdaten geben an, ob eine bestimmte Behandlungsfunktionalität der Behandlungseinrichtung aktiviert oder deaktiviert ist. Diese Zustandsdaten können dann von einem entfernt angeordneten Computer (beispielsweise bei einem auf Implantate spezialisierten Arzt) empfangen werden. Dann kann der Arzt bzw. ein anderer Benutzer des entfernt angeordneten Computers erkennen, welche Behandlungsfunktionalitäten von der implantierbaren Anordnung deaktiviert wurden, um zu entscheiden, ob eine Reaktivierung dieser Behandlungsfunktionalitäten im Interesse des Patienten ist. Die Zustandsdaten können dabei - wie auch die Reaktivierungsdaten in diesem oder in anderen Ausführungsbeispielen - verschlüsselt übertragen werden. Hierfür eignen sich die hinlänglich bekannten herkömmlichen Verschlüsselungsmechanismen.

In einer Variante ist es möglich, eine Reaktivierung einzelner Behandlungsfunktionalitäten zu gestatten, während eine Reaktivierung anderer Behandlungsfunktionalitäten nicht gestattet wird. Zu diesem Zweck können eine oder mehrere Behandlungsfunktionalitäten einer ersten Gruppe zugeordnet werden und eine oder mehrere andere Behandlungsfunktionalitäten einer zweiten Gruppe. Die beiden Gruppen lassen sich anschließend hinsichtlich der Reaktivierung unterschiedlich behandeln. Konkret veranlasst das Programm den Prozessor in dieser Variante dazu, eine Reaktivierung einer oder mehrerer Behandlungsfunktionalitäten, die der ersten Gruppe zugeordnet sind, auf der Grundlage der empfangenen Reaktivierungsdaten zu gestatten. Gleichzeitig veranlasst das Programm den Prozessor dazu, eine Reaktivierung einer einzelnen oder mehrerer Behandlungsfunktionalitäten, die der zweiten Gruppe zugeordnet sind, nicht zu gestatten. Das Vorsehen unterschiedlicher Gruppen erhöht somit die Patientensicherheit, da bestimmte Behandlungsfunktionalitäten, von denen eine besonders große Patientengefährdung ausgeht, somit gezielt von der Reaktivierung mittels Datenfernübertragung ausgeschlossen werden können. Zur Reaktivierung derartiger Funktionen wird weiterhin das Aufsuchen eines auf implantierbare Anordnungen spezialisierten Arztes erforderlich, der eine entsprechende Reaktivierung dann nach einer vorherigen Untersuchung des Patienten vornehmen kann.

Um eine Zuordnung einzelner oder mehrerer Behandlungsfunktionalitäten zu der ersten Gruppe oder zu der zweiten Gruppe vorzunehmen, verwendet der Prozessor in einer Variante Zuordnungsdaten. Diese Zuordnungsdaten können in der Speichereinrichtung der implantierbaren Anordnung hinterlegt sein oder der Anordnung auf anderem Wege übermittelt werden. Mittels derartiger Zuordnungsdaten ist eine flexible Zuordnung von Behandlungsfunktionalitäten zu der ersten Gruppe oder zu der zweiten Gruppe möglich. Beispielsweise können derartige Zuordnungsdaten vorsehen, dass eine bestimmte Behandlungsfunktionalität zu einem ersten Zeitpunkt der ersten Gruppe zuzuordnen ist. Zu einem späteren Zeitpunkt können derartige Zuordnungsdaten eine Zuordnung derselben Behandlungsfunktionalität in die zweite Gruppe veranlassen. Die Zuordnungsdaten können beispielsweise einen Gesundheitszustand des Patienten berücksichtigen, der eine erleichterte oder eine erschwerte Reaktivierung bestimmter Behandlungsfunktionalitäten ermöglicht bzw. erfordert.

In einer Variante ist eine Übermittlung von Zuordnungsdaten an die implantierbare Anordnung mittels der Datenfernübertragungseinrichtung ausgeschlossen.

In einer Variante weist die Anordnung eine Nahfeldtelemetrieeinrichtung auf. In dieser Variante veranlasst das Programm den Prozessor dazu, Zuordnungsdaten mittels der Nahfeldtelemetrieeinrichtung zu empfangen. Auf diese Weise ist es möglich, bestimmte Behandlungsfunktionalitäten für eine Reaktivierung freizuschalten oder von einer Reaktivierung auszunehmen, und zwar beispielsweise wenn sich der Patient bei einem nicht auf Implantate spezialisierten Arzt befindet, beispielsweise bei seinem Hausarzt. Dann kann der Hausarzt nicht unmittelbar eine Reaktivierung einzelner zuvor deaktivierter Behandlungsfunktionalitäten veranlassen. Es ist für ihn jedoch möglich, mittels der Nahfeldtelemetrieeinrichtung eine Freischaltung bestimmter Behandlungsfunktionalitäten vorzunehmen. Anschließend können dann Reaktivierungsdaten von einem Spezialisten an die implantierbare Anordnung gesendet und von dieser mittels der Datenfernübertragungseinrichtung empfangen werden. Somit würde eine doppelte Kontrolle eines Zugriffs auf die implantierbare Anordnung erfolgen. Einerseits muss mittels der Nahfeldtelemetrieeinrichtung eine Freischaltung bestimmter Behandlungsfunktionalitäten für eine Reaktivierung erfolgen. Andererseits muss ein auf Implantate spezialisierter Arzt oder entsprechend geschultes Fachpersonal zusätzlich Reaktivierungsdaten an die implantierbare Anordnung senden. Durch ein derartiges mehrstufiges Sicherheitsverfahren lässt sich eine Gefährdung durch einen unautorisierten Zugriff auf die implantierbare Anordnung signifikant reduzieren. Dennoch muss der Patient nur einen einzigen Arzt aufsuchen, bei dem es sich zudem um keinen Spezialisten für Implantate handeln muss.

Die mittels der Nahfeldtelemetrieeinrichtung oder auf anderem Wege an die implantierbare Anordnung gesendeten Zuordnungsdaten können beispielsweise eine temporäre Zuordnung bestimmter Behandlungsfunktionalitäten zur ersten Gruppe von Behandlungsfunktionalitäten ermöglichen. Dann kann es beispielsweise möglich sein, innerhalb eines Zeitfensters mit einer Dauer von beispielsweise 1 Minute bis 10 Stunden, insbesondere 5 Minuten bis 5 Stunden, insbesondere 10 Minuten bis 2 Stunden, insbesondere 20 Minuten bis 1,5 Stunden, insbesondere 30 Minuten bis 1 Stunde eine Reaktivierung der zuvor deaktivierten Behandlungsfunktionalität zu gestatten. Anschließend erfolgt eine automatische Zuordnung der entsprechenden Behandlungsfunktionalität zur zweiten Gruppe. Eine Reaktivierung der Behandlungsfunktionalität ist dann nicht mehr möglich. Wenn innerhalb des Zeitfensters keine Reaktivierung stattgefunden hat, müssen zunächst neue Zuordnungsdaten an die implantierbare Anordnung gesendet werden, um die grundsätzliche Möglichkeit einer Reaktivierung der deaktivierten Behandlungsfunktionalität erneut zu eröffnen.

In einer Variante handelt es sich bei der implantierbaren Anordnung um eine Anordnung zur Behandlung, insbesondere zur Stimulation, eines menschlichen oder tierischen Herzens. Beispielsweise kann die Anordnung ein Herzschrittmacher sein, der unterschiedliche Funktionalitäten aufweist. Die Anordnung kann beispielsweise eine implantierbare Anordnung zur Stimulation eines menschlichen oder tierischen Herzens sein, die dafür vorgesehen und eingerichtet ist, eine atriale anti-tachykarde Stimulation durchzuführen. Aber auch beliebige andere Funktionalitäten sind denkbar.

Die Anordnung kann in einer Variante aber auch ein Medizinprodukt sein, das für gänzlich andere diagnostische und/oder therapeutische Anwendungen innerhalb eines Patienten eingesetzt wird. Beispiele hierfür sind implantierbare Defibrillatoren, Neurostimulatoren, implantierbare Medikamentenpumpen und Herzunterstützungspumpen (Ventricular Assist Devices).

In einer Variante weist die Behandlungseinrichtung mindestens eine Behandlungsfunktionalität auf, die ausgewählt ist aus der Gruppe bestehend aus einer ventrikulären Stimulation, einer ventrikulären Konditionierungsstimulation, einer atrialen Stimulation, einer atrialen anti-tachykarden Stimulation und einer Defibrillation. Bei der ventrikulären Stimulation kann es sich beispielsweise um eine rechtsventrikuläre Overdrive-Stimulation, einer linksventrikuläre Overdrive-Stimulation oder eine biventrikuläre Overdrive-Stimulation handeln. Die biventrikuläre Overdrive-Stimulation kann dabei beispielsweise mit einer von einer regulären Stimulation abweichenden VV-Zeit ausgeführt werden. Spezifische Anpassungen der ventrikulären Konditionierungsstimulation an eine zuvor detektierte atriale Tachykardie, wie sie an anderer Stelle in der vorliegenden Anmeldung beschrieben werden, sind dabei ebenfalls denkbar.

Ein Aspekt der vorliegenden Erfindung bezieht sich auf eine Behandlungsanordnung, die eine implantierbare Anordnung zur diagnostischen und/oder therapeutischen Behandlung eines menschlichen oder tierischen Patienten gemäß den vorstehenden Erläuterungen sowie eine davon entfernt angeordnete Fernzugriffseinrichtung aufweist. Wie erläutert, weist die implantierbare Anordnung einen ersten Prozessor, eine erste Speichereinrichtung, eine Behandlungseinrichtung und eine erste Datenfernübertragungseinrichtung auf. Die Fernzugriffseinrichtung weist einen zweiten Prozessor, eine zweite Speichereinrichtung und eine zweite Datenfernübertragungseinrichtung auf. Die Fernzugriffseinrichtung kann ein gewöhnlicher Computer sein. Die erste Speichereinrichtung weist ein erstes computerlesbares Programm auf, das den ersten Prozessor dazu veranlasst, die nachfolgend erläuterten Schritte auszuführen, wenn es auf den ersten Prozessor ausgeführt wird.

Zunächst wird ermittelt, ob durch eine Behandlungsfunktionalität der Behandlungseinrichtung eine Gefährdung eines Patienten, dem die Anordnung implantiert wurde, dann auftreten könnte, wenn eine diagnostische und/oder therapeutische Behandlung des Patienten, die von der Behandlungsfunktionalität vorgegeben wird, durchgeführt würde.

Wenn eine derartige mögliche Patientengefährdung ermittelt wurde, wird die Behandlungsfunktionalität deaktiviert. Dann kann die durch die Behandlungsfunktionalität definierte Behandlung nicht mehr durchgeführt werden.

Wenn nachfolgend Reaktivierungsdaten mittels der ersten Datenfernübertragungseinrichtung empfangen werden, kann anschließend eine Reaktivierung der deaktivierten Behandlungsfunktionalität unter Berücksichtigung der empfangenen Reaktivierungsdaten erfolgen.

Die zweite Speichereinrichtung weist ein zweites computerlesbares Programm auf, das den zweiten Prozessor dazu veranlasst, die nachfolgend erläuterten Schritte auszuführen, wenn das Programm auf dem Prozessor ausgeführt wird.

Als Reaktion auf eine Eingabe oder eine Programmanforderung werden Reaktivierungsdaten erzeugt. Diese Reaktivierungsdaten werden anschließend mittels der zweiten Datenfernübertragungseinrichtung gesendet. Typischerweise erfolgen die Schritte des Erzeugens der Reaktivierungsdaten und des Sendens der Reaktivierungsdaten vor dem Schritt des Empfangs der Reaktivierungsdaten mittels der ersten Datenfernübertragungseinrichtung. Es ist dabei möglich und angedacht, dass die Reaktivierungsdaten bereits erzeugt sind und erst dann, wenn eine Reaktivierung einer bestimmten Behandlungsfunktionalität erforderlich wird, mittels der zweiten Datenfernübertragungseinrichtung gesendet werden, damit sie mit der ersten Datenübertragungseinrichtung empfangen werden können.

Eine Eingabe, die die Erzeugung von Reaktivierungsdaten veranlasst, kann beispielsweise eine Benutzereingabe an der Fernzugriffseinrichtung sein. Beispielsweise kann ein Arzt, nachdem er darüber informiert wurde, dass eine bestimmte implantierbare Anordnung eine Behandlungsfunktionalität deaktiviert hat, die nun reaktiviert werden soll, durch eine Eingabe an einer Benutzerschnittstelle einer Erzeugung von Reaktivierungsdaten veranlassen. Hierzu kann sich der Arzt zuvor telefonisch oder auf anderem Wege davon überzeugen, dass eine Reaktivierung der Behandlungsqualität keine Patientengefährdung mehr darstellt, beispielsweise weil sich zwischenzeitlich der Zustand des Patienten verändert hat.

In einer Variante veranlasst das zweite Programm den zweiten Prozessor dazu, vor dem erzeugende Reaktivierungsdaten mittels der zweiten Datenfernübertragungseinrichtung Zustandsdaten der implantierbaren Anordnung zu empfangen. Diese Zustandsdaten geben an, ob eine bestimmte Behandlungsfunktionalität der Behandlungseinrichtung aktiviert oder deaktiviert ist. Denn nur dann, wenn eine Behandlungsfunktionalität tatsächlich deaktiviert ist, müssen Reaktivierungsdaten in Bezug auf diese Behandlungsfunktionalität übersandt werden. Die Zustandsdaten können auch angeben, ob eine aus der Ferne erfolgende Reaktivierung für die jeweilige Behandlungsfunktionalität zulässig ist oder nicht.

Beispielsweise ist es möglich, dass ein mit der Reaktivierung einer Behandlungsfunktionalität beauftragter Arzt zunächst den Zustand der entsprechenden implantierbaren Anordnung überprüft. Dies kann auf der Grundlage solcher Zustandsdaten erfolgen, die von der ersten Datenfernübertragungseinrichtung an die zweite Datenfernübertragungseinrichtung gesendet werden. Wenn der Arzt dann erkennt, dass eine bestimmte Behandlungsfunktionalität deaktiviert wurde, ihm gleichzeitig aber Informationen vorliegen, dass eine Patientengefährdung aufgrund einer zwischenzeitlich erfolgten Änderung des Patientenzustandes nicht mehr gegeben ist, kann der Arzt die erforderlichen Reaktivierungsdaten in der Fernzugriffseinrichtung erzeugen. Eine derartige Änderung des Patientenzustandes kann beispielsweise durch eine medikamentöse Behandlung eingetreten sein.

In einer Variante ist vorgesehen, dass die grundsätzliche Möglichkeit der Reaktivierung einer Behandlungsfunktionalität nicht aus der Ferne geändert werden kann. Wenn eine Behandlungsfunktionalität also dahingehend markiert ist, dass sie nicht aus der Ferne reaktiviert werden kann, ist es für einen Arzt nicht möglich, mittels eines Fernzugriffs eine Änderung dieser Markierung dahingehend vorzunehmen, dass doch eine Reaktivierung mittels Fernzugriff ermöglicht wird. Vielmehr ist hierfür in einer Variante eine Interaktion mit der implantierbaren Anordnung in weitaus größerer Nähe, beispielsweise durch einen direkten Zugriff oder einen Zugriff über eine Nahfeldtelemetrieeinrichtung, erforderlich. Dadurch wird ein unautorisierter Zugriff auf die implantierbare Anordnung und den Aktivitätszustand der einzelnen Behandlungsfunktionalitäten signifikant erschwert.

Ein Aspekt, der jedoch nicht beansprucht wird, betrifft ein Verfahren zur Steuerung des Betriebs einer implantierbaren Anordnung zur diagnostischen und/oder therapeutischen Behandlung eines menschlichen oder tierischen Patienten gemäß den vorstehenden Erläuterungen. Dieses Verfahren weist die nachfolgend erläuterten Schritte auf.

Zunächst wird ermittelt, ob durch eine Behandlungsfunktionalität einer Behandlungseinrichtung der Anordnung eine Gefährdung eines Patienten, dem die Anordnung implantiert wurde, auftreten könnte, wenn eine durch die Behandlungsfunktionalität definierte diagnostische und/oder therapeutische Behandlung des Patienten durchgeführt würde.

Wenn eine solche mögliche Patientengefährdung erkannt wird, wird die Behandlungsfunktionalität deaktiviert.

Erst dann, wenn Reaktivierungsdaten mittels einer Datenfernübertragungseinrichtung empfangen wurden, kann nachfolgend eine Reaktivierung der deaktivierten Behandlungsfunktionalität auf der Grundlage der empfangenen Reaktivierungsdaten erfolgen. Werden derartige Reaktivierungsdaten hingegen nicht empfangen, verbleibt die Behandlungsfunktionalität in ihrem deaktivierten Zustand.

Ein Aspekt der vorliegenden Erfindung betrifft ein Computerprogrammprodukt mit einem computerlesbaren Code, der einen Prozessor dazu veranlasst, die nachfolgend erläuterten Schritte auszuführen, wenn er auf dem Prozessor ausgeführt wird.

Zunächst wird ermittelt, ob durch eine Behandlungsfunktionalität einer Behandlungseinrichtung einer implantierbaren Anordnung zur diagnostischen und/oder therapeutischen Behandlung eines menschlichen oder tierischen Patienten eine Gefährdung eines Patienten, dem die Anordnung implantiert wurde, dann auftreten könnte, wenn eine durch die Behandlungsfunktionalität definierte diagnostische und/oder therapeutische Behandlung des Patienten durchgeführt würde.

Wenn eine derartige mögliche Patientengefährdung ermittelt wurde, wird die Behandlungsfunktionalität deaktiviert.

Eine Reaktivierung der Behandlungsfunktionalität ist nur dann möglich, wenn Reaktivierungsdaten vorliegen. Diese Reaktivierungsdaten werden mittels einer Datenfernübertragungseinrichtung empfangen. Nach dem Empfang können die Reaktivierungsdaten dazu verwendet werden, die zuvor deaktivierte Behandlungsfunktionalität wieder zu reaktivieren. Werden keine Reaktivierungsdaten empfangen, verbleibt die Behandlungsfunktionalität in ihrem deaktivierten Zustand.

Ein Aspekt, der jedoch nicht beansprucht wird, betrifft ein Verfahren zur Behandlung eines menschlichen oder tierischen Patienten, der eine solche Behandlung benötigt. Diese Behandlung erfolgt mittels einer implantierbaren Anordnung zur diagnostischen und/oder therapeutischen Behandlung. Dabei weist die Anordnung einen Prozessor, eine Speichereinrichtung, eine Behandlungseinrichtung und eine Datenfernübertragungseinrichtung auf. Das Verfahren umfasst die nachfolgend näher erläuterten Schritte.

Zunächst wird ermittelt, ob durch eine Behandlungsfunktionalität der Behandlungseinrichtung eine Gefährdung eines Patienten, dem die Anordnung implantiert wurde, dann auftreten könnte, wenn eine durch die Behandlungsfunktionalität definierte diagnostische und/oder therapeutische Behandlung des Patienten durchgeführt würde.

Wenn eine derartige mögliche Patientengefährdung ermittelt wurde, wird die Behandlungsfunktionalität deaktiviert. Sie verbleibt in ihrem deaktivierten Zustand, bis sie auf der Grundlage von Reaktivierungsdaten reaktiviert wird.

Derartige Reaktivierungsdaten können mittels der Datenfernübertragungseinrichtung empfangen werden. Nach dem Empfang der Reaktivierungsdaten kann eine Reaktivierung der zuvor deaktivierten Behandlungsfunktionalität erfolgen.

Anschließend kann eine durch die Behandlungsfunktionalität definierte diagnostische und/oder therapeutische Behandlung an dem Patienten durchgeführt werden.

Ein Aspekt der vorliegenden Erfindung betrifft eine implantierbare Anordnung zur Stimulation eines menschlichen oder tierischen Herzens mit den nachfolgend erläuterten Merkmalen. Eine solche Anordnung ist insbesondere zur dauerhaften Implantation in einem menschlichen oder tierischen Patienten vorgesehen.

Solch eine implantierbare Anordnung weist einen Prozessor, eine Speichereinrichtung, eine Stimulationseinrichtung und eine erste Detektionseinrichtung zum Detektieren einer Herzrhythmusstörung einer Herzregion auf.

Erfindungsgemäß ist vorgesehen, dass die Speichereinrichtung ein computerlesbares Programm aufweist, das den Prozessor dazu veranlasst, die nachfolgend erläuterten Schritte auszuführen, wenn das Programm auf dem Prozessor ausgeführt wird.

Zunächst wird mittels der ersten Detektionsvorrichtung detektiert, ob in einer Herzregion eines Herzens eines menschlichen oder tierischen Patienten eine Herzrhythmusstörung vorliegt. Von einem Vorliegen einer Herzrhythmusstörung im Sinne der vorliegenden Erfindung ist dann auszugehen, wenn der Beginn oder die Präsenz einer Herzrhythmusstörung detektiert werden kann.

Falls eine solche Herzrhythmusstörung erkannt wurde, wird eine geeignete Stimulationsstrategie anhand eines Auswahlkriteriums ausgewählt. Das Auswahlkriterium umfasst dabei eine Messgröße oder eine aus der Messgröße berechnete Größe. Die Messgröße ist ausgewählt aus der Gruppe umfassend eine physiologische Messgröße des Patienten, eine pathophysiologische Messgröße des Patienten und eine nicht-physiologische Messgröße, die einen Zustand des Patienten angibt. Insbesondere besteht die zur Auswahl stehende Gruppe aus den vorstehend aufgelisteten Messgrößen oder einer Teilmenge davon. Das Auswahlkriterium kann mehr als eine dieser Messgrößen umfassen, die auch miteinander verrechnet werden können. Dabei ist eine unterschiedliche Gewichtung einzelner oder aller Messgrößen möglich.

Gemäß einer Ausführungsform wird der Status des Herzens vor der aATP-Abgabe erfasst. Der Status kann durch verschiedene Parameter abgebildet werden, die im Rahmen der Erfindung beschrieben werden. Auf Grundlage mindestens einer dieser Parameter kann ein geeigneter Zeitpunkt zur aATP-Therapieabgabe mit einer hohen Therapieerfolgswahrscheinlichkeit ermittelt werden.

Nachfolgend wird die Herzregion, in der die Herzrhythmusstörung erfasst wurde, mit der ausgewählten Stimulationsstrategie stimuliert. Hierzu wird die Stimulationseinrichtung verwendet. Bei der Stimulationseinrichtung kann es sich um eine atriale Stimulationseinrichtung oder um eine ventrikuläre Stimulationseinrichtung handeln. Dabei können ein Atrium oder beide Atrien und/oder ein oder beide Ventrikel durch die Stimulationseinrichtung stimuliert werden. Die Stimulation der Herzregion oder der Herzregionen wird mit dem Ziel durchgeführt, die detektierte Herzrhythmusstörung zu beenden. Bei der Herzrhythmusstörung kann es sich beispielsweise um eine atriale Tachykardie/Tachyarrhythmie (AT) oder eine atriale Fibrillation (AFib) handeln. Durch die ausgewählte Stimulationsstrategie wird im Idealfall eine Stimulation durchgeführt, die innerhalb einer kurzen Zeitspanne unter Einsatz einer möglichst geringen Energiemenge zu einer Terminierung der detektierten Herzrhythmusstörung wie etwa der detektierten AT/AFib führt und deren Terminierungserfolg langanhaltend ist, also bis zum erneuten Auftreten einer AT/AFib eine lange Zeitdauer verstreicht.

Anschließend werden ein Erfolg und/oder eine Effizienz der durchgeführten Stimulation erfasst. Dies kann mittels der ersten Detektionseinrichtung und/oder einer weiteren Detektionseinrichtung der Anordnung erfolgen.

Nun werden der erfasste Erfolg und/oder die detektierte Effizienz mit einem vorgebbaren Erfolgs- und/oder Effizienzkriterium verglichen. Auf diese Weise ist es möglich, zu bestimmen, ob ein erwünschter Erfolg bzw. eine erwünschte Effizienz erreicht werden konnte. Ein Erfolg einer Stimulation ist beispielsweise die Wiederherstellung eines normalen Herzrhythmus. Indikatoren für die Effizienz einer durchgeführten Stimulation sind beispielsweise die zur Wiederherstellung eines normalen Herzrhythmus erforderliche Zeit, die dafür benötigte Energie, oder auch die Zeit, die von der ersten AT/AFib Terminierung bis zum erneuten Auftreten einer AT/AFib vergeht. Verlängert sich diese Zeit für sukzessive AT/AFib, spricht dies für die Wirksamkeit der Stimulation gemäß der Erfindung.

Wenn das vorgebbare Erfolgs- und/oder Effizienzkriterium erreicht wurde, ist keine Anpassung der Stimulationsstrategie erforderlich. Vielmehr weist die gewählte Stimulationsstrategie dann für die behandelte Herzrhythmusstörung bereits einen hinreichend guten Erfolg bzw. eine hinreichend gute Effizienz auf. Wenn das vorgebbare Erfolgs- und/oder Effizienzkriterium hingegen nicht erreicht wurde, wird die Stimulationsstrategie optimiert, damit bei einer nachfolgenden Stimulation mit einer optimierten Stimulationsstrategie ein besserer Erfolg und/oder eine höhere Effizienz erreicht werden kann. Die Optimierung der Stimulationsstrategie umfasst dabei eine Veränderung der Stimulationsstrategie in Bezug auf mindestens einen Parameter. Dieser Parameter ist ausgewählt aus der Gruppe umfassend eine Form der Behandlung, eine Anzahl der Behandlungen, eine Kombination verschiedener Behandlungen, eine Häufigkeit der Behandlungen und einen Zeitpunkt der Behandlung. Insbesondere besteht die Gruppe aus den vorstehend aufgelisteten Parametern oder einer Teilmenge davon.

Unter dem Begriff "Form der Behandlung" wird insbesondere eine Auslegung und/oder ein Muster von elektrischen Impulsen verstanden, die im Rahmen einer Stimulation von der Stimulationseinrichtung abgegeben werden.

Der Zeitpunkt der Behandlung kann beispielsweise derart gewählt werden, dass ein besonders guter Erfolg oder eine besonders hohe Effizienz der Stimulation erwartet werden kann. Denn eine Stimulation, die während einer bestimmten Phase eines Herzzyklus abgegeben wird, kann grundsätzlich eine höhere Erfolgsrate zur Behandlung einer Herzrhythmusstörung aufweisen, als eine Stimulation, die zu einem anderen Zeitpunkt des Herzrhythmus abgegeben wird.

In einer Variante handelt es sich bei der Messgröße um eine hämodynamische Messgröße des Patienten, um eine Amplitude eines intrakardialen Elektrogramms, um eine Amplitude eines Farfield-Signals, um eine Amplitude eines Elektrokardiogrammsignals, um eine Amplitude eines atrialen Signals, um einen systemischen Blutdruck, um einen arteriellen Blutdruck, um einen venösen Blutdruck, um einen Blutdruck in einer der Herzkammern, um einen pulmonalarteriellen Blutdruck (PAP-Blutdruck), um einen sonstigen Blutdruck, um eine Änderung eines der vorgenannten Blutdrücke, um eine Änderung einer Morphologie eines erfassten Signals, um eine Impedanz-Änderung eines erfassten Signals, um einen Messwert, der einen Rückschluss auf eine Regelmäßigkeit oder Unregelmäßigkeit des Herzrhythmus des Patienten zulässt, um eine Körperlage des Patienten, um eine Kontraktilität des Herzmuskels, oder um die Veränderung der Kontraktilität des Herzmuskels. Alternativ oder zusätzlich kann die Messgröße auch eine Größe sein, die eine der vorgenannten Größen angibt bzw. widerspiegelt.

Eine Körperlage des Patienten kann beispielsweise eine aufrechte Lage, eine sitzende Lage oder einer liegende Lage (insbesondere eine waagerechte Lage) sein. Durch eine Auswahl einer oder mehrerer der vorgenannten Messgrößen ist es möglich, den aktuellen Zustand des Patienten bzw. das Befinden des Patienten bei der Auswahl einer geeigneten Stimulationsstrategie mit zu berücksichtigen. Wenn beispielsweise ein erhöhter Blutdruck des Patienten detektiert wird, wird eine Stimulationsstrategie ausgewählt, die nicht zu einer Blutdruckerhöhung führt, um den ohnehin schon erhöhten Blutdruck des Patienten nicht weiter zu erhöhen. In vergleichbarer Weise können andere physiologische Messgrößen des Patienten herangezogen werden, um die Stimulationsstrategie derart auszuwählen, dass eine Abweichung der jeweiligen Messgröße von

- einem Normwert bzw. Normbereich,
- einem vordefinierten Wert, und/oder
- einem für den Patienten individuellen Messwert (beispielsweise ein Mittelwert, ein Median usw.)

durch die ausgewählte Stimulationsstrategie nicht weiter verstärkt wird.

Insbesondere dann, wenn es sich bei der Messgröße um eine hämodynamische Messgröße des Patienten handelt, ist in einer Variante vorgesehen, dass die Messgröße von der implantierbaren Anordnung selbst mittels der ersten Detektionseinrichtung oder mittels einer weiteren Detektionseinrichtung ermittelt werden kann. Dann ist es nicht erforderlich, eine zusätzliche Sensoreinrichtung zur Erfassung der Messgröße vorzusehen.

In einer weiteren Variante ist eine derartige zusätzliche Sensoreinrichtung, die unabhängig von der implantierbaren Anordnung zur Stimulation des menschlichen oder tierischen Herzens ausgebildet ist, jedoch vorgesehen. Durch eine derartige zusätzliche Sensoreinrichtung können sich neben hämodynamischen Parametern besonders einfach auch andere physiologische Parameter des Patienten bestimmen lassen, sodass die möglichen physiologischen, pathophysiologischen oder nicht-physiologischen Messgrößen, die zur Auswahl der Messgröße zu Verfügung stehen, erweitert werden.

In einer Variante handelt es sich bei der Effizienz der Stimulation um eine physiologische Effizienz. Dabei ist insbesondere die Zeitdauer, die benötigt wird, um eine erkannte Herzrhythmusstörung mittels der ausgewählten Stimulationsstrategie erfolgreich zu behandeln, ein zur Bestimmung der physiologischen Effizienz geeignetes Maß. Alternativ oder zusätzlich kann es sich bei der Effizienz um eine nicht-physiologische Effizienz, nämlich eine Effizienz der für die Stimulation benötigten Energie handeln. Jede implantierbare Anordnung zur Stimulation des menschlichen oder tierischen Herzens weist eine Energiequelle auf, die eine endliche Energiemenge enthält. Die Lebensdauer derartiger Anordnungen hängt maßgeblich von der Energienutzung ab. Wenn der Energieverbrauch bei zumindest gleichwertigem Erfolg der durchgeführten Stimulationen gesenkt werden kann, wird die Lebensdauer der Stimulationsanordnung erhöht. Dies ist mit einem erheblichen Patientenkomfort verbunden, da dann ein chirurgischer Eingriff zum Austausch der Stimulationsanordnung bzw. der Energiequelle der Stimulationsanordnung erst zu einem späteren Zeitpunkt erforderlich ist.

In einer Variante werden Stimulationsstrategien, die sich für einen bestimmten Typus einer Herzrhythmusstörung in der Vergangenheit als besonders erfolgversprechend oder effizient erwiesen haben, höher priorisiert als andere Stimulationsstrategien. Dann werden diese höher priorisierten Stimulationsstrategien bei der Durchführung einer erneuten Stimulation bevorzugt ausgewählt, um die Stimulation durchzuführen. Auf diese Weise können voraussichtlich erfolgreiche Stimulationsstrategien vorrangig angewendet werden. Dies führt typischerweise zu einer Erhöhung des Erfolgs bzw. einer Steigerung der Effizienz der durchgeführten Stimulation. Zur Durchführung dieser Variante veranlasst das Programm den Prozessor dazu, das Auswahlkriterium, die angewendete Stimulationsstrategie und den damit erzielten Erfolg und/oder die damit erzielte Effizienz in der Speichereinrichtung zu speichern. Anschließend erfolgt eine Priorisierung der gespeicherten Stimulationsstrategie in Abhängigkeit des erzielten Erfolgs und/oder der erzielten Effizienz. Diese Speicherung kann beispielsweise in Form einer Ranking-Tabelle erfolgen, wobei Stimulationsstrategien, die in der Ranking-Tabelle weiter oben aufgeführt sind, eine höhere Priorisierung aufweisen als weiter unten aufgelistete Stimulationsstrategien und bei einer nachfolgenden Auswahl einer geeigneten Stimulationsstrategie bevorzugt ausgewählt werden. Andere Arten der Priorisierung bzw. Hierarchisierung der unterschiedlichen zur Verfügung stehenden Stimulationsstrategien sind ebenfalls denkbar.

Um eine voraussichtlich wenig erfolgversprechende oder wenig effiziente Behandlung eines Patienten gar nicht erst durchzuführen bzw. um die für die Stimulationen vorhandene Energie möglichst effizient einzusetzen, ist es einer Variante vorgesehen, eine Stimulationsstrategie mit einer niedrigen Priorisierung zumindest zeitweise ganz von den zur Auswahl stehenden Stimulationsstrategien auszuschließen. Beispielsweise ist es möglich, eine derartig niedrig priorisierte Stimulationsstrategie für eine erste Zeitdauer nicht als ausführbare Stimulationsstrategie anzubieten. Die erste Zeitdauer kann dabei beispielsweise wenige Minuten, Stunden oder 2-5 Tage betragen. Um einen derartigen zeitweisen oder dauerhaften Ausschluss einzelner Stimulationsstrategien durchzuführen, veranlasst das Programm den Prozessor in dieser Variante dazu, eine niedrig priorisierte Stimulationsstrategie für eine erste Zeitdauer von den für eine anstehende Stimulation ausführbaren Stimulationsstrategien auszuschließen.

In einer Variante handelt es sich bei der Stimulationseinrichtung um eine atriale Stimulationseinrichtung, also eine Stimulationseinrichtung, die für die Stimulation eines Atriums oder beider Atrien vorgesehen und eingerichtet ist. Insbesondere ist es in dieser Variante vorgesehen, dass die ausführbaren Stimulationsstrategien solche Strategien sind, mittels denen sich eine atriale anti-tachykarde Stimulation (atriale ATP) anwenden lässt. In einer Variante handelt es sich bei der durchzuführenden Stimulationsstrategie um eine Kardioversion, insbesondere durch eine atriale Behandlung.

In einer Variante ist die Anordnung zur Stimulation des Herzens dafür ausgelegt, bei der Wahl der atrialen Therapie die ventrikuläre Aktivität des Herzens zu berücksichtigen. Der Hintergrund ist, dass grundsätzliche Risiken dabei bestehen können, eine Stimulationstherapie in den Vorhöfen bei Vorliegen einer ventrikulären Tachykardie (VT) vorzunehmen. Speziell kann es im Fall einer VT zu einer sogenannten retrograden Überleitung kommen, d.h. die VT wird über das kardiale Reizleitungssystem zurück in den Vorhof geleitet und bewirkt eine Erregung des Vorhofs. Die durch die VT verursachte atriale Erregung kann durch eine antitachykarde Stimulationstherapie im Vorhof nicht terminiert werden. Daher soll das Schrittmacherimplantat eine solche Erregung nicht als eine durch atriale Stimulation terminierbare AT/AFib interpretieren. Für diesen Zweck muss die ventrikuläre Aktivität gemessen und bei der Entscheidung über die Anwendung, Art und Timing der atrialen Stimulationstherapie mit berücksichtigt werden. Dafür kann beispielsweise gemessen werden, ob eine hohe ventrikuläre Herzrate (d.h. eine VT) vorliegt. Für die Erkennung einer retrograden Überleitung können bekannte Methoden der Erkennung über ein EKG (intrakardial oder Oberflächen-EKG) angewandt werden. Zum Beispiel ist eine 2:1 retrograde Überleitung in einem Oberflächen-EKG dadurch erkennbar, dass P-Wellen nach jeder zweiten R-Zacke sichtbar sind. Daher ist in einer Ausführungsform der vorliegenden Anordnung zur Stimulation des Herzens dazu ausgebildet, das Risiko einer retrograden Überleitung einer ventrikulären Tachykardie auf die Vorhöfe zu bestimmen und die abzugebende Stimulation derart anzupassen, dass dieses Risiko möglichst gering gehalten wird. Zu diesem Zweck veranlasst das Programm den Prozessor dann, wenn die erfasste Herzrhythmusstörung eine atriale Tachykardie ist, dazu, zunächst einen ventrikulären Herzrhythmus (also eine ventrikuläre Aktivität) zu bestimmen. Auf der Basis des bestimmten ventrikulären Herzrhythmus wird dann das Risiko einer retrograden Überleitung einer ventrikulären Tachykardie in zumindest einem Atrium bewertet. Eine zur Behandlung der atrialen Tachykardie vorgesehene Stimulation wird dann unter Berücksichtigung des Risikos der retrograden Überleitung einer ventrikulären Tachykardie bei Bedarf zeitlich und/oder örtlich angepasst. Durch eine derartige Berücksichtigung der ventrikulären Aktivität kann die Abgabe einer atrialen Stimulation verhindert werden, wenn die atriale Erregung durch eine VT verursacht ist und somit nicht durch eine atriale Stimulation terminierbar ist. Wenn ein solcher Zustand erkannt wird, kann die erfindungsgemäße Anordnung zur Stimulation beispielsweise die durchzuführende atriale anti-tachykarde Stimulation nicht ausführen oder zeitlich verzögern.

Im Rahmen der Bewertung des ventrikulären Herzrhythmus in Bezug auf das Risiko der retrograden Überleitung werden dabei vorzugsweise Diskriminierungsmerkmale, wie z.B. das Verhältnis von atrialem zu ventrikulärem Rhythmus oder die ventrikuläre Signalmorphologie, abgeleitet und zur Anpassung der örtlichen und/oder zeitlichen Durchführung der atrialen anti-tachykarden Stimulation verwendet. Dabei ist es möglich, eine geplante atriale anti-tachykarde Stimulation gänzlich zu unterbinden, wenn das Risiko einer retrograden Überleitung einer ventrikulären Tachykardie als zu hoch bewertet wurde.

In einer Variante ist die Stimulationseinrichtung dafür vorgesehen und eingerichtet, die Stimulation als elektrische Stimulation oder als optische Stimulation auszuführen. Somit bietet die Stimulationseinrichtung unterschiedliche Möglichkeiten, die zuvor detektierte Herzrhythmusstörung zu behandeln. Welches physikalische Prinzip für die entsprechende Behandlung ausgewählt wird, kann beispielsweise unter Berücksichtigung der Schwere der detektierten Herzrhythmusstörung entschieden werden.

Ein Aspekt, der jedoch nicht beansprucht wird, betrifft ein Verfahren zur Steuerung des Betriebs einer implantierbaren Anordnung zur Stimulation eines menschlichen oder tierischen Herzens. Dabei ist insbesondere die Steuerung des Betriebs einer implantierbaren Anordnung gemäß den vorherigen Erläuterungen vorgesehen. Dieses Steuerungsverfahren umfasst die nachfolgend erläuterten Schritte.

Zunächst wird mittels einer ersten Detektionseinrichtung überprüft, ob in einer Herzregion eines Herzens eines menschlichen oder tierischen Patienten eine Herzrhythmusstörung vorliegt.

Anschließend wird eine geeignete Stimulationsstrategie ausgewählt. Dafür wird ein Auswahlkriterium verwendet, das eine Messgröße oder eine aus einer Messgröße berechnete Größe umfasst. Die Messgröße ist dabei eine physiologische Messgröße des Patienten, eine pathophysiologische Messgröße des Patienten und/oder eine nicht-physiologische Messgröße, die einen Zustand des Patienten angibt. Eine Kombination verschiedener Messgrößen ist dabei denkbar. Außerdem können die verschiedenen Messgrößen miteinander verrechnet und bei Bedarf auch unterschiedlich stark gewichtet werden.

Wenn eine geeignete Stimulationsstrategie ausgewählt wurde, wird mindestens ein Impuls zur Stimulation der Herzregion, in der die Herzrhythmusstörung erfasst wurde, in einer Stimulationseinrichtung erzeugt. Die Art, Form und Dauer des mindestens einen Impulses wird dabei anhand der ausgewählten Stimulationsstrategie entschieden. Diese definiert somit den zu erzeugenden Puls und gestaltet diesen weiter aus.

Nachfolgend werden Daten über einen Erfolg und/oder eine Effizienz einer durchgeführten Stimulation erfasst und zur weiteren Verarbeitung bereitgestellt. Diese Daten werden im Rahmen des Steuerungsverfahrens dann mit einem vorgebbaren Erfolgs- und/oder Effizienzkriterium verglichen. Wenn das vorgebbare Erfolgs- und/oder Effizienzkriterium erreicht wurde, ist kein weiterer Verfahrensschritt erforderlich. Wenn das vorgebbare Erfolgs- und/oder Effizienzkriterium hingegen nicht erreicht wurde, wird die Stimulationsstrategie optimiert. Diese Optimierung dient dazu, bei einer nachfolgenden Stimulation mit einer optimierten Stimulationsstrategie einen besseren Erfolg und/oder eine höhere Effizienz zu erreichen. Die Optimierung umfasst eine Veränderung der Stimulationsstrategie in Bezug auf mindestens einen Parameter. Bei diesen Parameter kann es sich beispielsweise um eine Form der Behandlung, eine Anzahl der Behandlungen, eine Kombination verschiedener Behandlungen, eine Häufigkeit der Behandlungen und/oder einen Zeitpunkt der Behandlung handeln. Dabei können die vorgenannten Parameter einzeln oder in beliebiger Kombination miteinander verwendet werden.

Ein Aspekt der vorliegenden Erfindung betrifft ein Computerprogrammprodukt mit einem computerlesbaren Code, der einen Prozessor dazu veranlasst, die nachfolgend erläuterten Schritte auszuführen, wenn der Code auf dem Prozessor ausgeführt wird.

Zunächst wird mittels einer ersten Detektionseinrichtung detektiert, ob in einer Herzregion eines Herzens eines menschlichen oder tierischen Patienten eine Herzrhythmusstörung vorliegt.

Dann wird anhand eines Auswahlkriteriums eine geeignete Stimulationsstrategie ausgewählt. Das Auswahlkriterium umfasst eine Messgröße oder eine aus einer Messgröße berechnete Größe. Bei der Messgröße kann es sich um eine physiologische Messgröße des Patienten, eine pathophysiologische Messgröße des Patienten und/oder eine nicht-physiologische Messgröße, die einen Zustand des Patienten angibt, handeln.

Nun veranlasst das Programm den Prozessor zur Stimulation der Herzregion, in der die Herzrhythmusstörung erfasst wurde. Diese Stimulation erfolgt mittels einer Stimulationseinrichtung, wobei die Art und Weise der Stimulation durch die ausgewählte Stimulationsstrategie definiert wird.

Nachfolgend werden ein Erfolg und/oder eine Effizienz der durchgeführten Stimulation erfasst.

Dieser erfasste Erfolg und/oder diese erfasste Effizienz werden dann mit einem vorgebbaren Erfolgs- und/oder Effizienzkriterium verglichen.

Wenn das Erfolgs- und/oder Effizienzkriterium - gegebenenfalls im Rahmen einer ebenfalls vorgebbaren Toleranz - erreicht wurde, ist kein weiterer Verfahrensschritt erforderlich. Wenn das vorgebbare Erfolgs- und/oder Effizienzkriterium hingegen nicht erreicht wurde, wird die Stimulationsstrategie optimiert. Dies dient dazu, bei einer nachfolgenden Stimulation mit einer optimierten Stimulationsstrategie einen besseren Erfolg und/oder eine höhere Effizienz zu erreichen. Die Optimierung umfasst dabei eine Veränderung der Stimulationsstrategie in Bezug auf mindestens einen Parameter. Bei dem Parameter kann es sich um eine Form der Behandlung, eine Anzahl der Behandlungen, eine Kombination verschiedener Behandlungen, eine Häufigkeit der Behandlungen und/oder einen Zeitpunkt der Behandlung handeln. Beliebige Kombinationen und Teilmengen der vorgenannten Parameter können dabei berücksichtigt werden.

Ein Aspekt der vorliegenden Erfindung betrifft ein Verfahren zur Behandlung eines menschlichen oder tierischen Patienten, der eine solche Behandlung benötigt. Dieses Verfahren wird mittels einer implantierbaren Anordnung zur Stimulation des Herzen des Patienten durchgeführt, wobei die Anordnung einen Prozessor, eine Speichereinrichtung, eine Stimulationseinrichtung und eine erste Detektionseinrichtung zum Detektieren einer Herzrhythmusstörung in einer Herzregion aufweist. Das Behandlungsverfahren umfasst die nachfolgend erläuterten Schritte.

Zunächst wird mittels der ersten Detektionseinrichtung detektiert, ob in einer Herzregion eines Herzen eines menschlichen oder tierischen Patienten eine Herzrhythmusstörung vorliegt.

Wenn eine solche Herzrhythmusstörung detektiert wurde, wird eine geeignete Stimulationsstrategie anhand eines Auswahlkriteriums ausgewählt. Das Auswahlkriterium umfasst eine Messgröße oder eine aus der Messgröße berechnete Größe. Die Messgröße ist dabei eine physiologische Messgröße des Patienten, eine pathophysiologische Messgröße des Patienten und/oder eine nicht-physiologische Messgröße, die einen Zustand des Patienten angibt. Es ist möglich, mehr als eine Messgröße vorzusehen, wobei beliebige Kombinationen der vorgenannten Messgrößen bzw. von Messgrößen aus diesen Messgrößengruppen eingesetzt werden können.

Nachfolgend wird die Herzregion, in der die Herzrhythmusstörung erfasst wurde, unter Anwendung der ausgewählten Stimulationsstrategie mittels der Stimulationseinrichtung stimuliert.

Anschließend werden ein Erfolg und/oder eine Effizienz der durchgeführten Stimulation detektiert. Dies kann mittels der ersten Detektionseinrichtung oder einer weiteren Detektionseinrichtung der implantierbaren Anordnung erfolgen.

Der detektierte Erfolg und/oder die detektierte Effizienz werden dann mit einem vorgebbaren Erfolgs- und/oder Effizienzkriterium verglichen.

Wenn das vorgebbare Erfolgs- und/oder Effizienzkriterium erreicht wurde, ist kein weiterer Verfahrensschritt erforderlich. Wenn das vorgebbare Erfolgs- und/oder Effizienzkriterium hingegen nicht erreicht wurde, wird die Stimulationsstrategie optimiert, damit bei einer nachfolgenden Stimulation mit einer optimierten Stimulationsstrategie ein besserer Erfolg und/oder eine höhere Effizienz erreicht werden kann. Die Optimierung umfasst eine Veränderung der Stimulationsstrategie in Bezug auf mindestens einen Parameter. Dieser Parameter ist ausgewählt aus der Gruppe umfassend eine Form der Behandlung, eine Anzahl der Behandlung, eine Kombination verschiedener Behandlungen, eine Häufigkeit der Behandlungen und einen Zeitpunkt der Behandlung. Die Gruppe kann insbesondere aus den vorstehend genannten Parametern bestehen. Beliebige Kombinationen und Untermengen der vorgenannten Parameter sind dabei ebenfalls denkbar.

Sämtliche Varianten und alternativen Ausgestaltungen, die in Zusammenhang mit den verschiedenen implantierbaren Anordnungen beschrieben wurden, sind beliebig miteinander kombinierbar und auf die jeweils anderen Anordnungen übertragbar. Sie sind in analoger Weise auch in beliebiger Kombination auf die beschriebenen Verfahren und die beschriebenen Computerprogrammprodukte übertragbar. Ferner sind die beschriebenen Varianten der Verfahren beliebig miteinander kombinierbar und auf die jeweils anderen Verfahren sowie auf die Computerprogrammprodukte und die Anordnungen übertragbar. In gleicher Weise sind die beschriebenen Varianten der Computerprogrammprodukte beliebig miteinander kombinierbar und auf die jeweils anderen Computerprogrammprodukte sowie auf die beschriebenen Verfahren und die beschriebenen Anordnungen übertragbar.

Weitere Details von Aspekten der Erfindung werden nachfolgend in Zusammenhang mit Ausführungsbeispielen und Zeichnungen näher erläutert. Es zeigen:

Figur 1      ein Blockschaltbild eines Ausführungsbeispiels einer implantierbaren Anordnung zur Stimulation des menschlichen oder tierischen Herzens;

Figur 2      einen schematischen Ablaufplan eines Ausführungsbeispiels einer atrialen antitachykarden Therapie;

Figur 3      eine schematische Darstellung der Anordnung mehrerer Elektroden in einem menschlichen Herz;

Figur 4      eine schematische Darstellung des zeitlichen Verlaufs eines Ausführungsbeispiels einer kardialen Behandlung;

Figur 5      ein Flussdiagramm eines Ausführungsbeispiels einer kardialen Behandlung;

Figur 6      ein vereinfachtes Blockschaltbild eines Ausführungsbeispiels einer Anordnung zur Behandlung des menschlichen oder tierischen Herzens;

Figur 7      ein Blockschaltbild eines Ausführungsbeispiels einer Behandlungsanordnung zur diagnostischen und/oder therapeutischen Behandlung eines Patienten;

Figur 8      ein Flussdiagramm eines schematischen Ablaufs eines beispielhaften Verfahrens, das von einem Ausführungsbeispiel einer implantierbaren Anordnung zur diagnostischen und/oder therapeutischen Behandlung eines Patienten durchgeführt wird;

Figur 9      ein Blockschaltbild eines Ausführungsbeispiels einer implantierbaren Anordnung zur Stimulation des menschlichen oder tierischen Herzens;

Figur 10      einen schematischen Ablaufplan eines Ausführungsbeispiels einer atrialen antitachykarden Therapie;

Figur 11      ein Blockschaltbild eines Ausführungsbeispiels einer implantierbaren Anordnung zur Stimulation des menschlichen oder tierischen Herzens;

Figur 12      einen schematischen Ablaufplan eines Ausführungsbeispiels der Detektion einer Herzrhythmusstörung und

Figur 13      einen schematischen Ablaufplan eines Ausführungsbeispiels einer Behandlung einer Herzrhythmusstörung mittels einer Stimulation einschließlich vorgeschalteter Detektion der Herzrhythmusstörung.

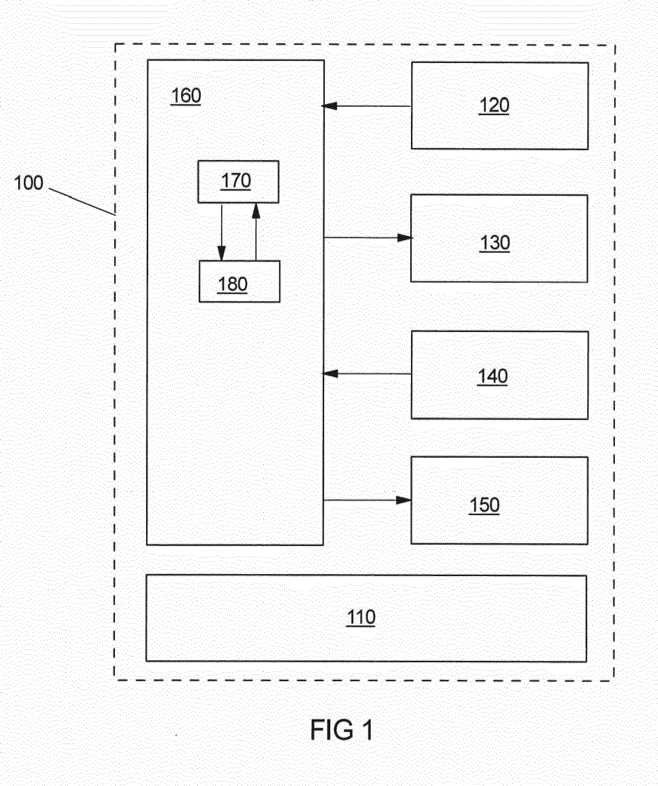

Die Figur 1 zeigt ein Blockschaltbild eines Ausführungsbeispiels eines Herzschrittmachers 100, der als implantierbare Anordnung zur Stimulation des menschlichen oder tierischen Herzens dient. Der Herzschrittmacher 100 weist eine Energiequelle 110, eine erste Detektionseinrichtung 120, eine atriale Stimulationseinrichtung 130, eine zweite Detektionseinrichtung 140 und eine ventrikuläre Stimulationseinrichtung 150 auf. Die erste Detektionseinrichtung 120, die atriale Stimulationseinrichtung 130, die zweite Detektionseinrichtung 140 und die ventrikuläre Stimulationseinrichtung 150 stehen in Wirkverbindung mit einer Steuereinrichtung 160. Der Steuereinrichtung 160 zugeordnet sind ein Prozessor 170 und ein Speicher 180. Im Speicher 180 sind Programminformationen enthalten, die den Prozessor 170 dazu veranlassen, bestimmte Schritte durchzuführen, wenn das Programm auf dem Prozessor 170 ausgeführt wird.

Diese Schritte sehen vor, dass von der ersten Detektionseinrichtung 120 erfasst wird, ob in einem menschlichen oder tierischen Herz eine zu behandelnde atriale Tachykardie vorliegt. Wenn eine solche behandlungspflichtige atriale Tachykardie erkannt wird, veranlasst der Prozessor 170 mittels der Steuereinrichtung 160 die ventrikuläre Stimulationseinrichtung 130 dazu, eine ventrikuläre Konditionierungsstimulation eines oder beider Herzventrikel durchzuführen. Optional wird der Effekt dieser ventrikulären Konditionierungsstimulation durch die zweite Detektionseinrichtung 140 erfasst und über die Steuereinrichtung 160 an den Prozessor 170 mitgeteilt. Während und/oder nach der Durchführung der ventrikulären Konditionierungsstimulation durch die ventrikuläre Stimulationseinrichtung 130 sorgt der Prozessor 170 zusammen mit der Steuereinrichtung 160 dafür, dass die atriale Stimulationseinrichtung 150 eine antitachykarde Stimulation des (rechten) Atriums des zu behandelnden Herzens durchführt.

Die für den Betrieb der einzelnen Komponenten des Herzschrittmachers 100 benötigte Energie wird durch die Energiequelle 110 bereitgestellt.

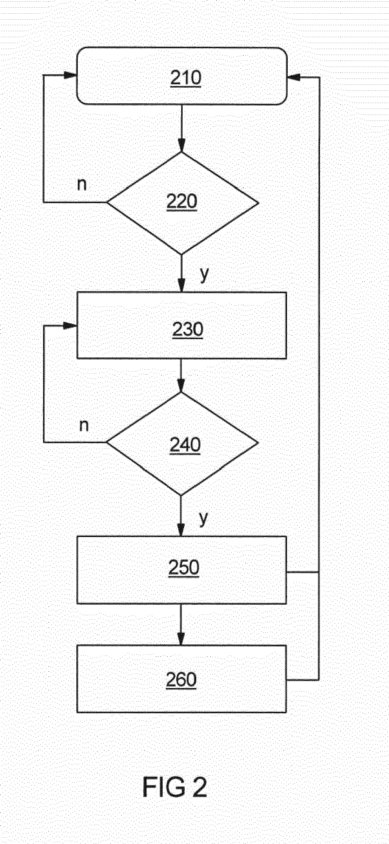

Die Figur 2 zeigt den schematischen Ablaufplan eines Ausführungsbeispiels einer atrialen Therapie, die beispielsweise mittels des Herzschrittmachers 100 der Figur 1 durchgeführt werden kann.

In einem ersten Schritt erfolgt eine kontinuierliche Rhythmusüberwachung 210 des atrialen Rhythmus des Patienten, der den Herzschrittmacher trägt. Wird im Rahmen dieser atrialen Rhythmusüberwachung 210 in einem Entscheidungsschritt 220 eine behandlungspflichtige atriale Tachykardie festgestellt, wird eine ventrikuläre Vorbehandlung 230 durchgeführt. Dabei bezeichnet in der Figur 2 und in allen weiteren Figuren der Buchstabe "n" eine negative Entscheidung bzw. ein negatives Ergebnis einer zuvor durchgeführten Überprüfung, während der Buchstabe "y" eine positive Entscheidung bzw. ein positives Ergebnis einer zuvor durchgeführten Überprüfung bezeichnet.

Die ventrikuläre Vorbehandlung 230 kann beispielsweise in Form einer ventrikulären Stimulation ausgeführt sein, die durch jede zweite atriale Erregung getriggert wird und mit einer kurzen AV-Zeit von beispielsweise 10 ms durchgeführt wird. Dann wird eine ventrikuläre Kontraktion bereits in einer Phase mit einer noch reduzierten ventrikulären Füllung initiiert. Die ventrikuläre Kontraktion wird somit unter verringerter Vorlast ausgeführt, sodass eine negativ inotrope Wirkung erzielt wird und kurzzeitig ein verringerter systemischer Blutdruck resultiert. Die derart erzeugte Blutdruckreduktion steigert den Therapieerfolg einer gleichzeitig oder nachfolgend durchgeführten atrialen ATP.

Die ventrikuläre Konditionierungsstimulation (ventrikuläre Vorbehandlung) wird zunächst bis zum Erreichen einer festgelegten Vorbehandlungszeit festgesetzt. Es wird also in einem Überprüfungsschritt 240 geprüft, ob die festgelegte Vorbehandlungszeit bereits erreicht ist. Ist dies der Fall, wird anschließend eine atriale ATP 250 abgegeben. Nach Abgabe der atrialen ATP 250 kann optional eine Post-ATP-Behandlung 260 durchgeführt werden. Alternativ kann der Herzschrittmacher unmittelbar nach Abgabe der atrialen ATP 250 wieder in die kontinuierliche Rhythmusüberwachung 210 zurückfallen bzw. zurückgesetzt werden.

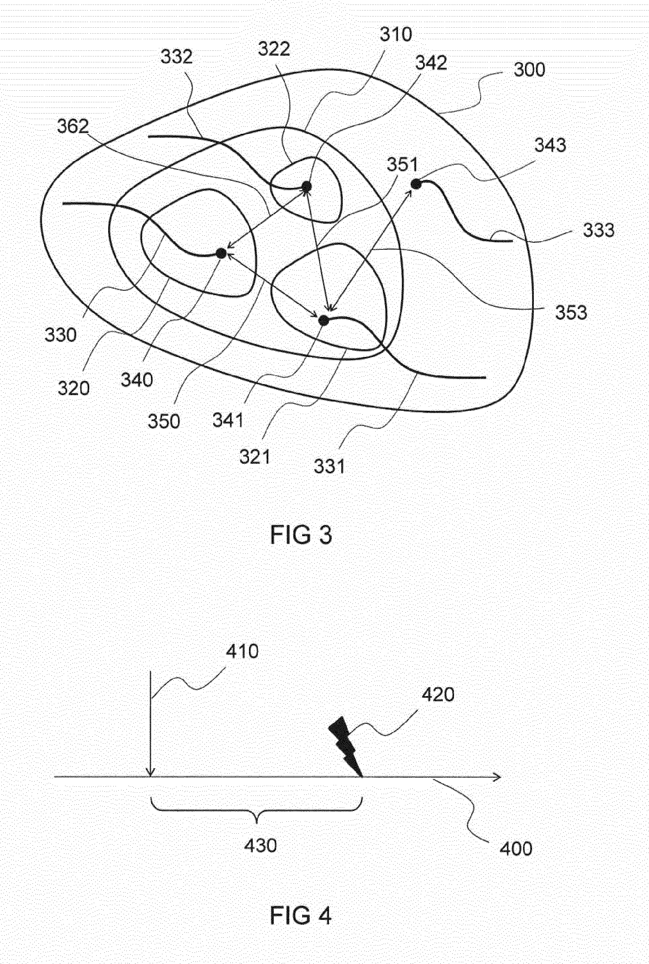

Die Figur 3 zeigt zur Verdeutlichung des Funktionsprinzips einer Elektrodenlagebestimmung einen Thorax 300 eines menschlichen Patienten, dem ein Herzschrittmacher als Anordnung zur Behandlung des Herzens implantiert wurde. Das Herz 310 ist schematisch in einem mittleren Bereich des Thorax 300 dargestellt. Das Herz 310 weist unterschiedliche Herzregionen 320, 321 und 322 auf. Hierbei kann es sich beispielsweise um den linken oder rechten Ventrikels oder um ein Atrium des Herzens 310 handeln. In der ersten Herzregion 320 ist eine erste Elektrode 330 angeordnet. In der zweiten Herzregion 321 in eine zweite Elektrode 331 angeordnet. Schließlich ist in der dritten Herzregion 322 eine dritte Elektrode 332 angeordnet. Die erste Elektrode 330, die zweite Elektrode 331 und die dritte Elektrode 332 weisen jeweils einen endständig angeordneten leitenden Abschnitt 340, 341 und 342 auf, der auch als Elektrodenpol bezeichnet werden kann.

Darüber hinaus ist außerhalb des Herzens 310 im Bereich des Thorax 300 eine vierte Elektrode 333 angeordnet, die terminal ebenfalls einen leitenden Abschnitt 343 aufweist.

Nun kann ein erster Abstand 350 zwischen dem ersten Elektrodenpol 340 der ersten Elektrode 330 und dem zweiten Elektrodenpol 341 der zweiten Elektrode 331 bestimmt werden. In gleicher Weise kann ein zweiter Abstand 351 zwischen dem zweiten Elektrodenpol 341 der zweiten Elektrode 331 und dem dritten Elektrodenpol 342 der dritten Elektrode 332 bestimmt werden. Darüber hinaus kann ein dritter Abstand 352 zwischen dem ersten Elektrodenpol 340 der ersten Elektrode 330 und dem dritten Elektrodenpol 342 der dritten Elektrode 332 bestimmt werden. Ebenso ist es möglich, einen Abstand zwischen jedem der Elektrodenpole 340, 341, 342 zum Referenzelektrodenpol 343 der Referenzelektrode 342 zu bestimmen. Ein solcher vierter Abstand 353 ist in der Figur 3 beispielhaft lediglich für den Abstand zwischen dem zweiten Elektrodenpol 341 der zweiten Elektrode 331 und dem Referenzelektrodenpol 343 der Referenzelektrode 333 dargestellt.

Nur dann, wenn die Abstände 350, 351, 352 und/oder 353 innerhalb vorgebbarer Bereiche liegen, werden die erste Elektrode 330, die zweite Elektrode 331 und/oder die dritte Elektrode 332 für eine kardiale Behandlung verwendet. Ist demgegenüber einer der ermittelten Abstände 350, 351, 352 und/oder 353 zu gering oder zu groß, wird eine kardiale Behandlung zunächst nicht durchgeführt, da sich zumindest eine Elektrode in einer inadäquaten Lage befindet.

Die Figur 4 zeigt in schematischer Weise den zeitlichen Ablauf einer kardialen Behandlung, bei der zunächst die Lage einer Behandlungselektrode überprüft wird. Dabei sind die stattfindenden Ereignisse auf einer Zeitachse 400 dargestellt. Ein Therapietrigger 410 plant eine spezifische kardiale Therapie 420, die auch als kardiale Behandlung bezeichnet werden kann. Dabei wird diese kardiale Therapie 420 durch den Therapietrigger 410 deswegen geplant, weil zuvor mittels einer Detektionseinrichtung ein kardiales Ereignis festgestellt wurde, dass als behandlungspflichtig eingestuft wurde. Vor Abgabe der kardialen Therapie 420 wird in einer Zeitspanne 430 jedoch überprüft, ob sich die für die kardiale Therapie 420 benötigten Elektroden (vergleiche hierzu die Figur 3) in einer für die kardiale Therapie 420 adäquaten Lage befinden. Nur wenn dies der Fall ist, wird die kardiale Therapie 420 tatsächlich durchgeführt. Andernfalls wird die Durchführung der kardialen Therapie 420 jedenfalls zunächst unterbunden.

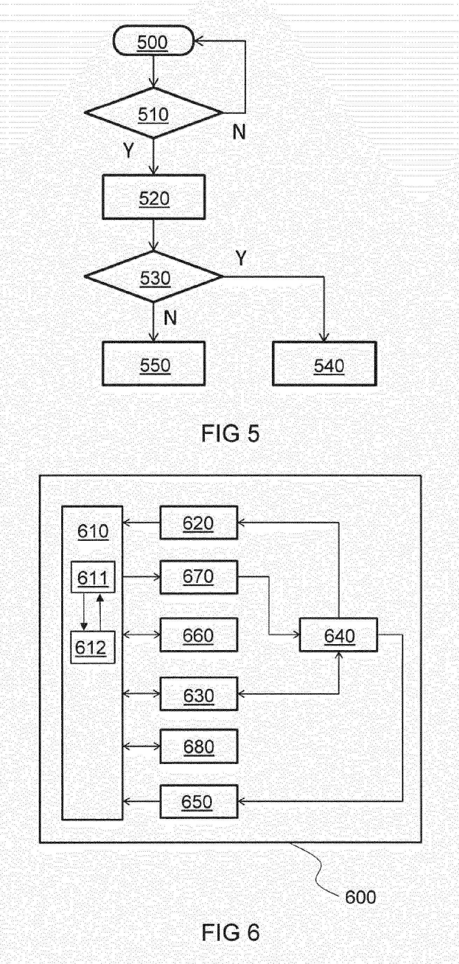

Die Figur 5 zeigt ein Flussdiagramm eines schematischen Ablaufs eines Verfahrens zur Behandlung eines menschlichen oder tierischen Herzens, bei dem vor Durchführung einer kardialen Therapie eine korrekte Elektrodenpositionierung überprüft wird. In einem ersten Schritt erfolgt eine kontinuierliche Rhythmusüberwachung 500 des Herzrhythmus des Patienten. Wird hierbei ein behandlungspflichtiges kardiales Ereignis festgestellt, wird nach einem ersten Entscheidungsschritt 510 eine Lagebestimmung 520 mindestens einer Behandlungselektrode eines Herzschrittmachers eingeleitet. Im Rahmen dieser Lagebestimmung 520 wird ermittelt, ob die Lage der Behandlungselektrode mit einer vorgebbaren Referenzlage übereinstimmt.

Wird in einem zweiten Entscheidungsschritt 530 festgestellt, dass die Lage mit der Referenzlage übereinstimmt, kann eine kardiale Behandlung 540 unter Einsatz der Behandlungselektrode durchgeführt werden. Wird demgegenüber in dem zweiten Entscheidungsschritt 530 ermittelt, dass die festgestellte Lage der Behandlungselektrode nicht mit der erwarteten Lage übereinstimmt, wird der Herzschrittmacher in einen Wartezustand 550 überführt. Aus diesem Wartezustand 550 kann der Herzschrittmacher wieder in die kontinuierliche Rhythmusüberwachung 500 überführt werden. Alternativ ist es auch möglich, eine kardiale Behandlung beispielsweise manuell auszulösen.

Die Figur 6 zeigt ein vereinfachtes Blockschaltbild eines implantierbaren Herzschrittmachers 600, der eine Lagebestimmung mindestens einer Behandlungselektrode ausführt. Der Herzschrittmacher 600 dient als implantierbare Anordnung zur Behandlung eines menschlichen oder tierischen Herzens. Er weist eine Steuereinrichtung 610 auf, die mit einem Prozessor 611 und einer Speichereinrichtung 612 ausgestattet ist. Eine Detektionseinrichtung 620 überprüft, ob in dem menschlichen oder tierischen Herz des Patienten, dem der Herzschrittmacher 600 implantiert wurde, ein zu behandelndes kardiales Ereignis eingetreten ist. Das Ergebnis dieser Überprüfung übermittelt die Detektionseinrichtung 620 an die Steuereinrichtung 610.

Wenn ein zu behandelndes kardiales Ereignis eingetreten ist, veranlasst der Prozessor 611 über die Steuereinrichtung 610 eine Lageüberprüfungseinrichtung 630, die Lage einer Behandlungselektrode 640 zu überprüfen. Zu diesem Zweck vergleicht die Lageüberprüfungseinrichtung 630 die festgestellte Lage der Elektrode 640 mit einer Referenzlage. Dabei können zusätzliche Daten, die über die Lage der Behandlungselektrode 640 Aufschluss geben, mittels eines zusätzlichen Lagesensors oder Beschleunigungssensors 650 herangezogen werden.

Die von der Lagebestimmungseinrichtung 630 und optional von den zusätzlichen Sensoren 650 bereitgestellten Daten werden in einer Analyseeinrichtung 660 ausgewertet. Ergibt sich dabei, dass die Lage der Behandlungselektrode 640 einer für die anstehende kardiale Therapie adäquaten Lage entspricht, steuert der Prozessor 611 über die Steuereinrichtung 610 eine Therapieeinrichtung 670 an, die eine zur Behandlung des kardialen Ereignisses adäquate Therapie mittels der Behandlungselektrode 640 an die entsprechende Herzregion abgibt.

Die von der Lagebestimmungseinrichtung 630 erfassten Daten zur Lage der Behandlungselektrode 640 können zusammen mit einer Zeitinformation in einem Trendspeicher 680 gespeichert werden, um einen Trend der Lagerveränderung der Behandlungselektrode 640 auswerten bzw. erkennen zu können.

Wenn die Analyseeinrichtung 660 eine inadäquate Lage der Behandlungselektrode 640 feststellt, wird die Therapieeinrichtung 670 nicht zur Abgabe einer kardialen Behandlung angesteuert. Vielmehr wird dann zunächst keine kardiale Behandlung durchgeführt.

Die Behandlungselektrode 640 kann zudem gleichzeitig als Detektionselektrode wirken und somit bereits zusammen mit der Detektionseinrichtung 620 bei der Detektion eines zu behandelnden kardialen Ereignisses eingesetzt werden.

Nicht alle der in dem Blockschaltbild der Figur 6 dargestellten Einrichtungen müssen notwendigerweise separate Einheiten innerhalb des Herzschrittmachers 600 bilden. Vielmehr können zahlreiche Aufgaben von dem Prozessor 611 übernommen werden, wenn dieser entsprechende Programminformationen aus dem Speicher 612 erhält.

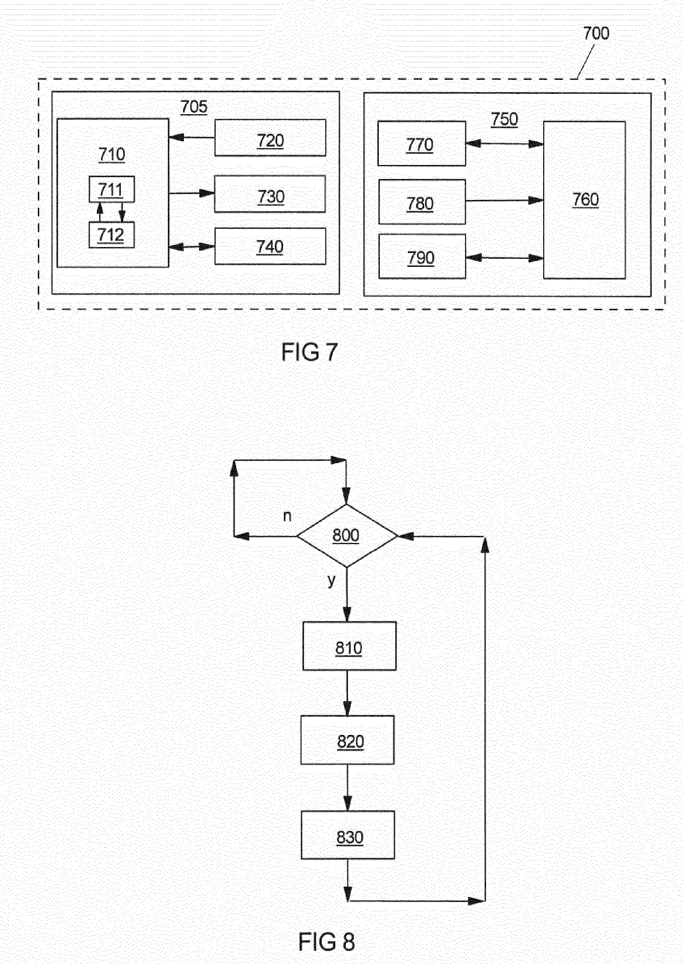

Die Figur 7 zeigt ein Ausführungsbeispiel einer Behandlungsanordnung 700 mit einem Implantat 705, das als implantierbare Anordnung zur diagnostischen und/oder therapeutischen Behandlung eines menschlichen oder tierischen Patienten dient. Das Implantat 705 weist eine erste Steuereinrichtung 710 mit einem ersten Prozessor 711 und einer ersten Speichereinrichtung 712 auf. Ferner ist eine Detektionseinrichtung 720 vorgesehen, die einen bestimmten physiologischen Parameter eines Patienten, dem das Implantat 700 implantiert wird, überprüft. Beispielsweise kann mit der Detektionseinrichtung 720 ein Herzrhythmus des Patienten überwacht werden. Darüber hinaus weist das Implantat 700 eine Behandlungseinrichtung 730 auf, mit der eine diagnostische und/oder therapeutische Behandlung des Patienten erfolgen kann. Diese Behandlungseinrichtung 730 weist eine Vielzahl unterschiedlicher Behandlungsfunktionalitäten auf, die jeweils eine bestimmte Behandlung definieren. Dabei kann eine Behandlungsfunktionalität auch eine Abfolge bestimmter diagnostischer und/oder therapeutischer Behandlungen vorgeben.

Das Implantat 705 weist zudem eine erste Datenfernübertragungseinrichtung 740 auf, mit der Daten von der Steuereinrichtung 710 bzw. dem Prozessor 711 gesendet und/oder empfangen werden können. Die Datenfernübertragungseinrichtung 740 arbeitet dabei vorzugsweise drahtlos.

Die Behandlungsanordnung 700 umfasst zudem eine Fernzugriffseinrichtung 750, die entfernt von dem Implantat 705 angeordnet ist. Beispielsweise kann die Fernzugriffseinrichtung 750 in einem Krankenhaus oder in der Praxis eines Arztes aufgestellt sein. Mittels der Fernzugriffseinrichtung 750 ist es möglich, in Kommunikationskontakt mit dem Implantat 705 zu treten. Zu diesem Zweck weist die Fernzugriffseinrichtung 750 einen zweiten Prozessor 760, eine zweite Speichereinrichtung 770, eine Benutzerschnittstelle 780 und eine zweite Datenfernübertragungseinrichtung 790 auf.

Der zweite Prozessor 760 kann aus der zweiten Speichereinrichtung 770 Programminformationen aufrufen, um ein entsprechendes Programm anschließend durchzuführen. Mittels der Benutzerschnittstelle 780 ist es möglich, Eingaben zur weiteren Verarbeitung von Daten durch den zweiten Prozessor 760 vorzunehmen. Beispielsweise kann ein Arzt mittels der Benutzerschnittstelle 780 manuell eine Reaktivierung von einer zuvor deaktivierten Behandlungsfunktionalität veranlassen. Der zweite Prozessor 760 wird dann über die zweite Datenfernübertragungseinrichtung 790 Reaktivierungsdaten an die erste Datenübertragungseinheit 740 des Implantats 705 senden. Wenn der erste Prozessor 711 des Implantats 705 derartige Reaktivierungsdaten erhält, kann er diese an die Behandlungseinrichtung 730 weitergeben und eine zuvor deaktivierte Behandlungsfunktionalität der Behandlungseinrichtung 730 wieder reaktivieren.

Das Implantat 705 und die Fernzugriffseinrichtung 750 können einige Meter, einige Kilometer, aber auch Hunderte oder Tausende von Kilometern entfernt voneinander angeordnet sein. Durch die Auswahl geeigneter Datenübertragungsprotokolle kann dennoch eine zuverlässige Kommunikation zwischen der im Implantat 705 angeordneten ersten Datenübertragungseinrichtung 740 und der in der für Fernzugriffseinrichtung 750 angeordneten zweiten Datenfernübertragungseinrichtung 790 gewährleistet werden.

Die Figur 8 zeigt ein Flussdiagramm zur schematischen Darstellung eines beispielhaften Ablaufs eines Verfahrens, das von einem Ausführungsbeispiel einer implantierbaren Anordnung zur diagnostischen und/oder therapeutischen Behandlung eines menschlichen oder tierischen Patienten ausgeführt werden kann, wie beispielsweise vom Implantat 705 der Figur 7.

Dabei bezieht sich dieser Ablaufplan auf die spezifische Anwendung im Bereich der atrialen anti-tachykarden Stimulation. Das heißt, es wird im Rahmen des in der Figur 8 dargestellten Ausführungsbeispiels davon ausgegangen, dass es sich bei dem Implantat um einen Herzschrittmacher, also eine Anordnung zur Stimulation des menschlichen oder tierischen Herzens, handelt.

In einem ersten Schritt 800 wird überprüft, ob die Abgabe einer atrialen anti-tachykarden Therapie aktiviert ist. Eine solche Abgabe einer atrialen anti-tachykarden Therapie stellt eine Behandlungsfunktionalität dar, die die Behandlungs- bzw. Stimulationseinrichtung des entsprechenden Implantats ausführen kann. Wird bei dieser Überprüfung ermittelt, dass die Abgabe einer atrialen anti-tachykarden Therapie freigegeben ist, kann eine solche atriale anti-tachykarde Therapie 810 abgegeben werden.

Nach Ablauf einer voreinstellbaren Zeit, die im hier beschriebenen Ausführungsbeispiel 48 Stunden beträgt, wird die weitere Abgabe einer atrialen anti-tachykarden Stimulation unterbunden. Denn wenn über einen derart langen Zeitraum eine atriale anti-tachykarde Stimulation erforderlich ist, steigt aufgrund der noch nicht erfolgreich behandelten atrialen Tachykardie das Thromboserisiko des Patienten. Der Patient muss sich nun bei seinem Hausarzt zu einer hausärztlichen Untersuchung 820 einfinden. Im Rahmen dieser Untersuchung erhält der Patient eine medikamentöse Antikoagulationstherapie. Durch die Gabe von antikoagulierenden Medikamenten wird das Thromboserisiko des Patienten wieder gesenkt. Diese Information, dass der Patient eine derartige Antikoagulationstherapie erhalten hat, wird an einen Implantationsarzt, also einen Arzt, der auf Implantate wie Herzschrittmacher spezialisiert ist, weitergegeben. Dieser Implantationsarzt kann dann im Rahmen einer Freigabe 830 mittels eines Fernzugriffs die Möglichkeit der Abgabe einer atrialen anti-tachykarden Stimulation wieder reaktivieren. Dazu sendet der Arzt Freigabedaten an den Herzschrittmacher des Patienten, die zu einer Reaktivierung der deaktivierten Behandlungsfunktionalität führen.

In der Folge können dann abermals atriale anti-tachykarde Stimulationen abgegeben werden, sofern dies aufgrund des Herzzustandes des Patienten noch erforderlich sein sollte. Falls sich der atriale Herzrhythmus des Patienten zwischenzeitlich wieder normalisiert haben sollte, bleibt die Möglichkeit der Abgabe atrialer anti-tachykarder Stimulationen dennoch aktiviert. Bei einem erneuten Auftreten einer atrialen Tachykardie kann dann eine entsprechende atriale anti-tachykarde Behandlung erfolgen.

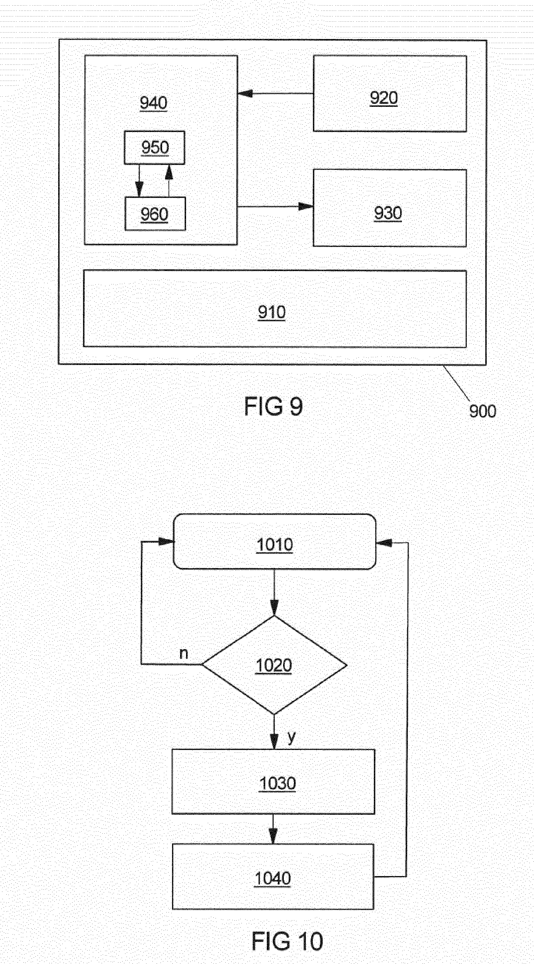

Die Figur 9 zeigt ein Blockschaltbild eines Ausführungsbeispiels eines Herzschrittmachers 900, der als implantierbare Anordnung zur Stimulation des menschlichen oder tierischen Herzens dient. Der Herzschrittmacher 900 weist eine Energiequelle 910 auf, mit der die einzelnen Komponenten des Herzschrittmachers 900 mit elektrischer Energie versorgt werden. Darüber hinaus weist der Herzschrittmacher 900 eine Detektionseinrichtung 920, eine atriale Stimulationseinrichtung 930 und eine Steuereinrichtung 940 auf, die sowohl mit der Detektionseinrichtung 920 als auch mit der atrialen Stimulationseinrichtung 930 in Wirkverbindung steht. Der Steuereinrichtung 940 sind ein Prozessor 950 und eine Speichereinrichtung 960 zugeordnet, die untereinander in Wirkverbindung stehen. In der Speichereinrichtung 960 sind Programminformationen enthalten, die den Prozessor 950 dazu veranlassen, bestimmte Schritte durchzuführen, wenn das Programm auf dem Prozessor 950 ausgeführt wird.

Im konkreten Fall des Ausführungsbeispiels der Figur 9 ruft der Prozessor 950 Programminformationen aus der Speichereinrichtung 960 ab, die ihn dazu veranlassen, zunächst Informationen von der Detektionsvorrichtung 920 darüber abzufragen, ob in einem menschlichen oder tierischen Herz eine atriale Tachykardie detektiert wurde. Wenn dies der Fall ist, veranlasst der Prozessor die atriale Stimulationseinrichtung 930 dazu, eine atriale anti-tachykarde Stimulation des betroffenen Atriums durchzuführen. Anschließend veranlasst der Prozessor 950 die atriale Stimulationseinrichtung 930 dazu, eine atriale Nachbehandlungsstimulation durchzuführen, um den Therapieeffekt der durchgeführten atrialen anti-tachykarden Stimulation zu erhöhen. Dazu kann der Prozessor 950 zunächst weitere Informationen von der Detektionsvorrichtung 920 darüber abrufen, ob die zuvor detektierte atriale Tachykardie durch die bereits durchgeführte atriale anti-tachykarde Stimulation beendet wurde. Er kann die Durchführung der atrialen Nachbehandlungsstimulation mittels der atrialen Stimulationseinrichtung 930 so lange aussetzen, bis seitens der Detektionsvorrichtung 920 positive Informationen über eine Terminierung der zuvor detektierten atrialen Tachykardie ermittelt wurden.

Die Figur 10 zeigt einen schematischen Ablaufplan eines Ausführungsbeispiels einer atrialen Therapie, die beispielsweise mittels des Herzschrittmachers 900 der Figur 9 durchgeführt werden kann.

In einem ersten Schritt erfolgt eine kontinuierliche Rhythmusüberwachung 1010 des atrialen Rhythmus eines Patienten, der den Herzschrittmacher trägt. Wird im Rahmen dieser atrialen Rhythmusüberwachung 1010 in einem Entscheidungsschritt 1020 festgestellt, dass eine behandlungspflichtige atriale Tachykardie vorliegt, wird nachfolgend eine atriale anti-tachykarde Stimulation 1030, die auch als atriale ATP bezeichnet werden kann, durchgeführt bzw. abgegeben. Anschließend erfolgt eine atriale Nachbehandlung 1040, die auch als ATP-Nachbehandlung bezeichnet werden kann. Diese Nachbehandlung wird mit einer geringeren Stimulationsfrequenz als die atriale anti-tachykarde Stimulation 1030 durchgeführt, weist jedoch immer noch eine größere Frequenz als die normale (intrinsische) atriale Herzfrequenz auf.

Nach Abschluss der atrialen Nachbehandlungsstimulation 1040 wird der Herzschrittmacher wieder in einen Status der kontinuierlichen Rhythmusüberwachung 1010 zurückversetzt. Dabei kann die Entscheidung, wann die atriale Nachbehandlungsstimulation 1040 beendet wird, beispielsweise in Abhängigkeit einer verstrichenen Zeit oder in Abhängigkeit einer bestimmten Anzahl an Herzzyklen des Patienten getroffen werden.

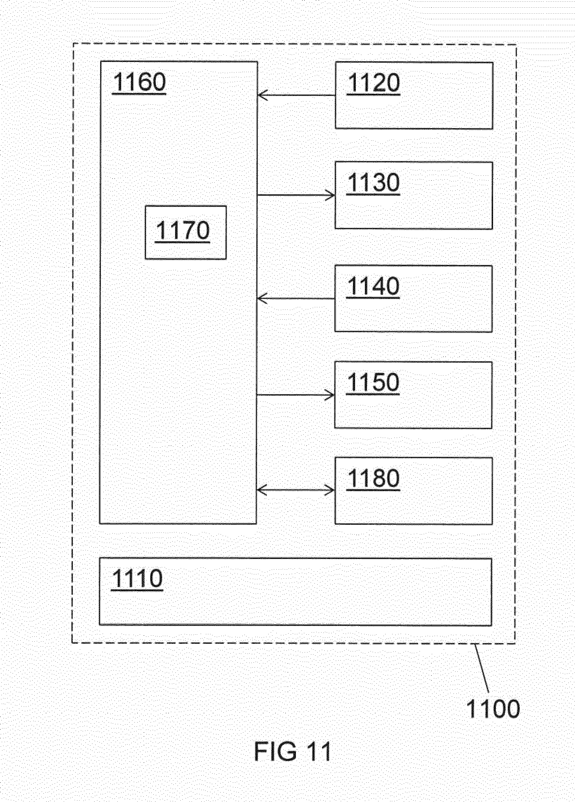

Die Figur 11 zeigt ein Blockschaltbild eines Herzschrittmachers 1100, der als implantierbare Anordnung zur Stimulation des menschlichen oder tierischen Herzens dient. Der Herzschrittmacher 1100 weist eine Energiequelle 1110, eine atriale Detektionseinrichtung 1120, eine atriale Stimulationseinrichtung 1130, eine ventrikuläre Detektionseinrichtung 1140, eine ventrikuläre Stimulationseinrichtung 1150 sowie eine Steuereinrichtung 1160 auf. Dabei sind der Steuereinrichtung 1160 ein Prozessor 1170 und ein Datenspeicher 1180 zugeordnet. Der Prozessor 1170 kann Daten aus dem Datenspeicher 1180 abrufen, beispielsweise damit ein Programm auf dem Prozessor 1170 ausgeführt werden kann. Die atriale Detektionseinrichtung 1120, die atriale Stimulationseinrichtung 1130, die ventrikuläre Detektionseinrichtung 1140 und die ventrikuläre Stimulationseinrichtung 1150 stehen in Wirkverbindung mit der Steuereinrichtung 1160, sodass der Prozessor Signale von den einzelnen Einrichtungen empfangen bzw. an die einzelnen Einrichtungen senden kann.

Im Datenspeicher 1180 kann ein Erfolg bzw. eine Effizienz einer zuvor mittels der atrialen Stimulationseinrichtung 1130 oder der ventrikulären Stimulationseinrichtung 1150 durchgeführten Stimulation gespeichert werden. Dabei werden typischerweise auch die der entsprechenden Stimulation zu Grunde liegende Stimulationsstrategie sowie die zuvor von der atrialen Detektionseinrichtung 1120 und/oder der ventrikulären Detektionseinrichtung 1140 detektierte Herzrhythmusstörung gespeichert. Darüber hinaus ist es möglich, zu den einzelnen Datensätzen aus detektierter Herzrhythmusstörung, angewendeter Stimulationsstrategie und erzieltem Erfolg bzw. erreichter Effizienz ein Prioritätskriterium zu vergeben und mit den Datensätzen abzuspeichern. Auf diese Weise kann Datensätzen, die sich auf eine besonders erfolgreiche oder besonders effiziente Stimulation beziehen, eine höhere Priorität zugewiesen werden.

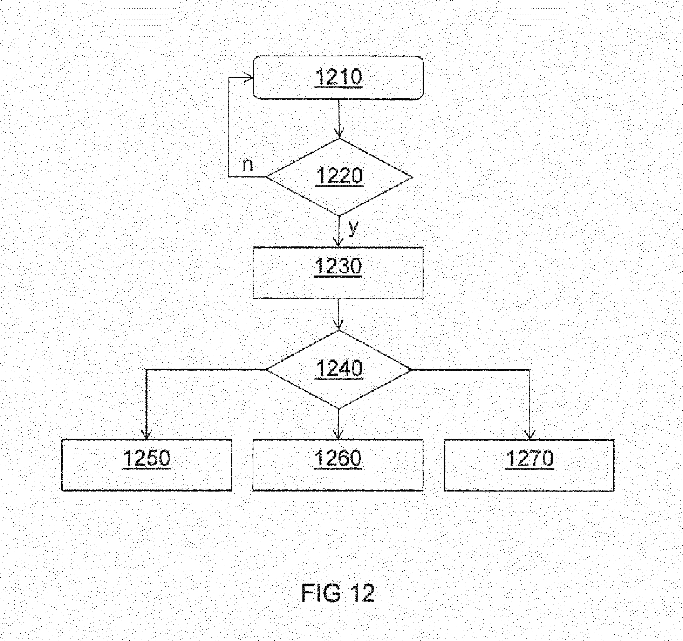

Die Figur 12 zeigt einen schematischen Ablaufplan für ein Verfahren, mit dem unterschiedliche Herzrhythmusstörungen voneinander unterschieden werden können. Dieses Verfahren kann von einem Herzschrittmacher, beispielsweise dem Herzschrittmacher 1100 der Figur 11 durchgeführt werden.

In einem ersten Schritt 1210 erfolgt eine kontinuierliche Rhythmusüberwachung des atrialen Herzrhythmus des Patienten, dem der entsprechende Herzschrittmacher implantiert wurde. Wenn im Rahmen dieser kontinuierlichen Rhythmusüberwachung 1210 in einem Entscheidungsschritt 1220 eine atriale Tachyarrhythmie oder eine atriale Tachykardie festgestellt wird, erfolgt zusätzlich eine Überwachung 1230 des ventrikulären Rhythmus des Patienten.

In einem nachfolgenden Entscheidungsschritt 1240 erfolgt eine genauere Analyse des detektierten atrialen Rhythmus und des detektierten ventrikulären Rhythmus in Bezug auf den resultierenden Herzrhythmus und die atrioventrikuläre Überleitung (AV-Überleitung). Dabei wird der resultierende Herzrhythmus in drei Rhythmuskategorien 1250, 1260 und 1270 eingeteilt. Die erste Rhythmuskategorie 1250 umfasst eine Präsenz einer VT oder auch einer VT mit retrograder Überleitung. Die zweite Rhythmuskategorie 1260 umfasst eine atriale Tachykardie/Tachyarrhythmie (AT) sowie eine atriale Fibrillation (AFib). Die dritte Rhythmuskategorie 1270 umfasst eine anterograde Überleitung der atrialen Tachykardie/Tachyarrhythmie sowie eine supraventrikuläre Tachykardie (SVT).

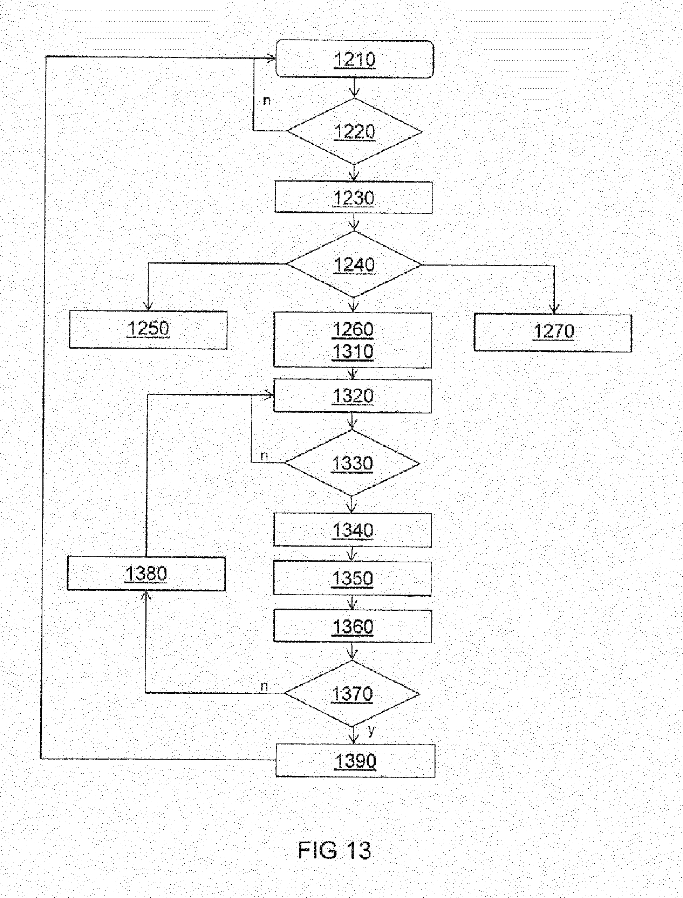

Nur dann, wenn ein resultierender Herzrhythmus der zweiten Kategorie 1260 detektiert wurde, also ein Herzrhythmus, der eine atriale Tachykardie/Tachyarrhythmie bzw. eine atriale Fibrillation umfasst, wird nachfolgend eine Stimulationsstrategie ausgewählt, die eine atriale anti-tachykarde Stimulation umfasst. Die Details der Abgabe dieser atrialen anti-tachykarden Stimulation sind in der Figur 13 näher dargestellt.

So zeigt die Figur 13 in ihrem oberen Teil zunächst die Verfahrensschritte, die bereits aus der Figur 12 bekannt sind. Hierbei werden die bereits in der Figur 12 verwendeten Bezugszeichen abermals verwendet. Hinsichtlich einer detaillierten Beschreibung wird auf die vorstehende Beschreibung der Figur 12 verwiesen.

Wenn bei der Kategorisierung des resultierenden Herzrhythmus ein Herzrhythmus der zweiten Kategorie 1260 identifiziert wird, also ein Herzrhythmus, der eine atriale Tachykardie bzw. eine atriale Fibrillation umfasst, erfolgt eine Aktivierung 1310 eines Ablaufplans für eine atriale anti-tachykarde Stimulation.

Zunächst erfolgt eine Ermittlung 1320 mindestens einer Messgröße, nämlich einer physiologischen Messgröße des Patienten und/oder einer pathophysiologischen Messgröße des Patienten und/oder einer nicht-physiologischen Messgröße, die einen Zustand des Patienten angibt. Dabei kann sich beispielsweise um eine Messgröße handeln, die die Körperlage des Patienten spezifiziert. Diese Messgröße oder einer aus dieser Messgröße berechnete Größe würde dann dazu verwendet, ein Auswahlkriterium zu bilden. Dieses Auswahlkriterium berücksichtigt im weitesten Sinne das Befinden des Patienten.

Anschließend wird in einem Überprüfungsschritt 1330 überprüft, ob der Zustand bzw. das Befinden des Patienten die grundsätzlichen Voraussetzungen für eine Stimulation erfüllt. Es wird also überprüft, ob für das gebildete Auswahlkriterium geeignete Stimulationsstrategien vorhanden sind. Falls dies nicht der Fall ist, wird (zunächst) keine Stimulation durchgeführt. Es können dann weitere Messgrößen erfasst werden, um den Zustand des Patienten noch genauer zu charakterisieren und ein neues Auswahlkriterium zu bilden.

Wenn die Messgröße oder die Messgrößen, die für das Auswahlkriterium herangezogen wurden, ergeben, dass der Patient die für die anstehende atriale anti-tachykarde Stimulation erforderlichen Voraussetzungen erfüllt, werden in einem nachfolgenden Auswahlschritt 1340 die im internen Datenspeicher des Herzschrittmachers verfügbaren Überstimulationstherapien abgerufen. Aus den verfügbaren Überstimulationstherapien bzw. Stimulationsstrategien werden anschließend in einem weiteren Auswahlschritt 1350 diejenigen Stimulationsstrategien ausgewählt, die den im Ermittlungsschritt 1320 ermittelten Bedingungen bzw. Messgrößen des Patienten am besten entsprechen. Um zu ermitteln, welche Stimulationsstrategien der zuvor ermittelten Messgröße am besten entsprechen, werden insbesondere die Form, die Auslegung und die Zusammensetzung der verfügbaren Stimulationsstrategien berücksichtigt. Darüber hinaus kann die Erfolgsrate bei den vorherigen Anwendungen der Stimulationsstrategien berücksichtigt werden. Die ausgewählte(n) Stimulationsstrategie(n) werden nachfolgend in einem Stimulationsabgabeschritt 1360 an den Patienten abgegeben, wobei sich die Reihenfolge der abgegebenen Stimulationen nach deren vorheriger Priorisierung ergibt.

Anschließend wird in einem weiteren Überprüfungsschritt 1370 überprüft, ob die durchgeführte(n) Stimulation(en) zu einer Terminierung der atrialen Tachykardie geführt hat. Sofern dies nicht der Fall ist, erfolgt in einem Optimierungsschritt 1380 eine Veränderung der bereits durchgeführten Stimulationsstrategie bzw. der ausgewählten noch durchzuführenden Stimulationsstrategien. Diese Anpassung erfolgt auf der Grundlage eines Parameters wie beispielsweise der Behandlungsform, der Behandlungsanzahl, der Kombination verschiedener Behandlung, der Behandlungshäufigkeit und des Behandlungszeitpunkts. Dabei wird in dem internen Datenspeicher des Herzschrittmachers auch vermerkt, dass die nicht angepasste Stimulationsstrategie nicht erfolgreich war. Ihr Prioritätswert wird in diesem Zusammenhang erniedrigt. Das heißt, dass die Priorität dieser Stimulationsstrategie herabgesetzt wird. Demgegenüber kann der Prioritätswert der angepassten (also optimierten) Stimulationsstrategie zunächst unverändert bleiben. Wenn ermittelt wurde, dass durch die optimierte Stimulationsstrategie eine erfolgreiche Terminierung der zuvor detektierten atrialen Tachykardie möglich ist, kann das Prioritätskriterium der entsprechenden Stimulationsstrategie erhöht werden. Dann wird diese Stimulationsstrategie bei einer späteren Behandlung bevorzugt angewendet.

Wenn im Entscheidungsschritt 1370 schließlich festgestellt wurde, dass eine Terminierung der atrialen Tachykardie erfolgt ist, wird dieser Erfolg zusammen mit den Details der angewendeten Stimulationsstrategie (insbesondere Stimulationsform, Stimulationsauslegung und Stimulationszusammensetzung) und dem zugrundeliegenden Auswahlkriterium bzw. den das Auswahlkriterium definierenden Messgrößen im internen Datenspeicher des Herzschrittmachers abgelegt. Zusätzlich wird dort die Information gespeichert, dass diese Stimulationsstrategie erfolgreich war. Außerdem wird dieser Stimulationsstrategie ein höherer Prioritätswert zugewiesen. Dies erfolgt im Speicherschritt 1390. Anschließend wird der Herzschrittmacher wieder in einen Zustand der kontinuierlichen Rhythmusüberwachung 1210 zurückversetzt.

Durch eine geeignete Kategorisierung einer detektierten Herzrhythmusstörung, eine Auswahl geeigneter Stimulationsstrategien anhand von Messgrößen, die in Zusammenhang mit dem Zustand des Patienten stehen sowie eine Priorisierung und Optimierung der verschiedenen Stimulationsstrategien kann im Ergebnis eine äußerst effiziente Behandlung von Herzrhythmusstörungen durchgeführt werden. Dabei erfordert diese Behandlung einen weitaus geringeren Energiebedarf als die aus dem Stand der Technik bekannten Behandlungen. Denn durch eine Optimierung und Priorisierung verschiedener Stimulationsstrategien werden Stimulationsstrategien, die für die jeweils detektierte Herzrhythmusstörung wenig erfolgversprechend sind, überhaupt nicht zur Anwendung gebracht. Dies reduziert den Energieaufwand des entsprechenden Herzschrittmachers und verlängert somit dessen Lebensdauer.

**Patentansprüche**

1. Implantierbare Anordnung zur Stimulation eines menschlichen oder tierischen Herzens, aufweisend einen Prozessor (950), eine Speichereinrichtung (960), eine atriale Stimulationseinrichtung (930) und eine Detektionseinrichtung (920) zum Detektieren einer atrialen Tachykardie, wobei die Speichereinrichtung (960) ein computerlesbares Programm aufweist, das den Prozessor (950) dazu veranlasst, die folgenden Schritte auszuführen, wenn es auf dem Prozessor (950) ausgeführt wird:

   a) Erfassen (210) mittels der Detektionseinrichtung (920), ob in einem menschlichen oder tierischen Herz eine zu behandelnde atriale Tachykardie vorliegt,
   b) wenn eine zu behandelnde atriale Tachykardie vorliegt, Ausführen einer atrialen anti-tachykarden Stimulation (1030) mittels der atrialen Stimulationseinrichtung (920),
   c) nach dem Ausführen der atrialen anti-tachykarden Stimulation (1030) und nur, wenn keine tachykarde intrinsische atriale Aktivität vorliegt, Ausführen einer atrialen Nachbehandlungsstimulation (1040), wobei die Nachbehandlungsstimulation (1040) im Bereich von 1 Minute bis zu 7 Tagen ausgelegt ist und mit einer geringeren Stimulationsfrequenz als die atriale anti-tachykarde Stimulation (1030) durchgeführt wird.

2. Implantierbare Anordnung nach Anspruch 1, wobei das Programm den Prozessor (950) dazu veranlasst, nach dem Ausführen der atrialen anti-tachykarden Stimulation (1030) zunächst mittels der Detektionseinrichtung (920) zu überprüfen, ob die atriale Tachykardie beendet ist und die atriale Nachbehandlungsstimulation (1040) nur dann auszuführen, wenn eine Beendigung der atrialen Tachykardie festgestellt wurde.

3. Implantierbare Anordnung nach Anspruch 1 oder 2, wobei das Programm den Prozessor (950) dazu veranlasst, die atriale Nachbehandlungsstimulation (1040) mit einer Stimulationsfrequenz von kleiner gleich 200 bpm, 180 bpm, 150 bpm, 140 bpm, 130 bpm, 120 bpm, 110 bpm, oder 100 bpm abzugeben.

4. Implantierbare Anordnung nach einem der vorhergehenden Ansprüche, wobei das Programm den Prozessor (950) dazu veranlasst, die atriale Nachbehandlungsstimulation (1040) als rechtsatriale Overdrive-Stimulation auszuführen.

5. Implantierbare Anordnung nach einem der Ansprüche 1 bis 3, wobei das Programm den Prozessor (950) dazu veranlasst, die atriale Nachbehandlungsstimulation (1040) als linksatriale Overdrive-Stimulation auszuführen.

6. Implantierbare Anordnung nach einem der Ansprüche 1 bis 3, wobei das Programm den Prozessor (950) dazu veranlasst, die atriale Nachbehandlungsstimulation (1040) als biatriale Overdrive-Stimulation auszuführen.

7. Implantierbare Anordnung nach einem der vorhergehenden Ansprüche, wobei das Programm den Prozessor (950) dazu veranlasst, die atriale Nachbehandlungsstimulation (1040) während einer ersten Zeitdauer auszuführen.

8. Implantierbare Anordnung nach einem der vorhergehenden Ansprüche, wobei das Programm den Prozessor (950) dazu veranlasst, die atriale Nachbehandlungsstimulation (1040) während einer definierten Anzahl an Herzzyklen auszuführen.

9. Implantierbare Anordnung nach einem der vorhergehenden Ansprüche, wobei das Programm den Prozessor (950) dazu veranlasst, die atriale Nachbehandlungsstimulation (1040) derart auszuführen, dass der Druck im linken und/oder rechten Atrium zumindest kurzzeitig gesenkt wird, dass das Risiko einer Mitralklappenregurgitation zumindest kurzzeitig reduziert oder vermieden wird, dass die Vorlast des Herzens zumindest kurzzeitig reduziert wird, dass der Blutdruck eines Patienten, dessen Herz durch die Anordnung stimuliert wird, zumindest kurzzeitig gesenkt wird, und/oder dass eine myokardiale Sauerstoffbilanz zumindest kurzzeitig verbessert wird, in dem eine für den Patienten als optimal ermittelte AV-Zeit und/oder eine biventrikuläre Stimulation und/oder eine erhöhte ventrikulären Stimulationsfrequenz eingestellt wird.

10. Implantierbare Anordnung nach einem der vorhergehenden Ansprüche, wobei die atriale Stimulationseinrichtung (930) dazu ausgelegt ist, die atriale anti-tachykarde Stimulation (1030) und/oder die atriale Nachbehandlungsstimulation (1040) als elektrische Stimulation oder als optische Stimulation auszuführen.

11. Computerprogrammprodukt mit einem computerlesbaren Code, der einen Prozessor (950) dazu veranlasst, die folgenden Schritte auszuführen, wenn er auf dem Prozessor (950) ausgeführt wird:

a) Erfassen (1010) mittels einer Detektionseinrichtung (920), ob in einem menschlichen oder tierischen Herz eine zu behandelnde atriale Tachykardie vorliegt,
b) wenn eine zu behandelnde atriale Tachykardie vorliegt, Ausführen einer atrialen anti-tachykarden Stimulation (1030) mittels einer atrialen Stimulationseinrichtung (920),
c) nach dem Ausführen der atrialen anti-tachykarden Stimulation (1030) und nur, wenn keine tachykarde intrinsische atriale Aktivität vorliegt, Ausführen einer atrialen Nachbehandlungsstimulation (1040), wobei die Nachbehandlungsstimulation (1040) im Bereich von 1 Minute bis zu 7 Tagen ausgelegt ist ist und mit einer geringeren Stimulationsfrequenz als die atriale anti-tachykarde Stimulation (1030) durchgeführt wird.

**Claims**

1. An implantable arrangement for stimulating a human or animal heart, the implantable system comprising a processor (950), a memory means (960), an atrial stimulator (930) and a detector for detecting atrial tachycardia,
wherein
the memory means (960) comprises a computer-readable program that prompts the processor (950) to carry out the following steps when said program is being executed on the processor (950):

a) detecting (210) by means of the detector (920) whether an atrial tachycardia to be treated is present in a human heart or animal heart;
b) performing an atrial antitachycardia stimulation (1030) by means of the atrial stimulator (920) when an atrial tachycardia to be treated is present;
c) performing an atrial post-treatment stimulation (1040) after the atrial antitachycardia stimulation (1030) has been performed and only when no tachycardiac intrinsic atrial activity is present, wherein the post-treatment stimulation (1040) is configured to be within a range of from 1 minute up to 7 days and is carried out at a lower stimulation rate than is used when performing the atrial antitachycardia stimulation (1030).

2. The implantable arrangement according to claim 1, wherein the program prompts the processor (950), after the atrial antitachycardia stimulation (1030) has been performed, to initially check by means of the detector (920) whether the atrial tachycardia has been terminated, and to carry out the atrial post-treatment stimulation (1040) only when a termination of the atrial tachycardia has been established.

3. The implantable arrangement according to claim 1 or 2, wherein the program prompts the processor (950) to deliver the atrial post-treatment stimulation (1040) using a stimulation rate of less than or equal to 200 bpm, 180 bpm, 150 bpm, 140 bpm, 130 bpm, 120 bpm, 110 bpm or 100 bpm.

4. The implantable arrangement according to any one of the preceding claims, wherein the program prompts the processor (950) to carry out the atrial post-treatment stimulation (1040) in a form of right atrial overdrive stimulation.

5. The implantable arrangement according to any one of claims 1 to 3, wherein the program prompts the processor (950) to carry out the atrial post-treatment stimulation (1040) in a form of left atrial overdrive stimulation.

**6.** The implantable arrangement according to any one of claims 1 to 3, wherein the program prompts the processor (950) to carry out the atrial post-treatment stimulation (1040) in a form of biatrial overdrive stimulation.

**7.** The implantable arrangement according to any one of the preceding claims, wherein the program prompts the processor (950) to carry out the atrial post-treatment stimulation (1040) during a first duration.

**8.** The implantable arrangement according to any one of the preceding claims, wherein the program prompts the processor (950) to carry out the atrial post-treatment stimulation (1040) during a defined number of cardiac cycles.

**9.** The implantable arrangement according to any one of the preceding claims, wherein the program prompts the processor (950) to carry out the atrial post-treatment stimulation (1040) in such a way that the pressure in the left and/or right atrium is lowered at least briefly, the risk of mitral valve regurgitation is reduced at least briefly or is avoided, the preload of the heart is reduced at least briefly, the blood pressure of a patient whose heart is being stimulated by the arrangement is lowered at least briefly, and/or a myocardial oxygen balance is improved at least briefly, by setting an AV delay ascertained as being optimal for the patient and/or a biventricular stimulation and/or an increased ventricular stimulation rate.

**10.** The implantable arrangement according to any one of the preceding claims, wherein the atrial stimulator (930) is configured to apply the atrial antitachycardia stimulation (1030) and/or the atrial post-treatment stimulation (1040) in a form of electrical stimulation or in a form of optical stimulation.

**11.** A computer program product comprising a computer-readable code that prompts a processor (950) to perform the following steps when said code is being executed on the processor (950):

a) detecting (1010) by means of a detector (920) whether an atrial tachycardia to be treated is present in a human heart or animal heart;
b) performing an atrial antitachycardia stimulation (1030) by means of an atrial stimulator (920) when an atrial tachycardia to be treated is present;
c) performing an atrial post-treatment stimulation (1040) after the atrial antitachycardia stimulation (1030) has been performed and only when no tachycardiac intrinsic atrial activity is present, wherein the post-treatment stimulation (1040) is configured to be within a range of from 1 minute up to 7 days and is carried out at a lower stimulation rate than is used when performing the atrial antitachycardia stimulation (1030).

**Revendications**

**1.** Ensemble implantable destiné à la stimulation d'un coeur humain ou animal, présentant un processeur (950), un dispositif de mémoire (960), un dispositif de stimulation atriale (930) et un dispositif de détection (920) permettant la détection d'une tachycardie atriale,
dans lequel
le dispositif de mémoire (960) présente un programme lisible par ordinateur qui fait en sorte que le processeur (950) exécute les étapes suivantes, lorsqu'il est effectué sur le processeur(950) :

a) la détection (210), au moyen du dispositif de détection (920), s'il y a une tachycardie atriale cardiaque à traiter dans un coeur humain ou animal,
b) si une tachycardie atriale à traiter est présente, l'exécution d'une stimulation anti-tachycardique atriale (1030) au moyen du dispositif de stimulation atriale (920),
c) après l'exécution de la stimulation anti-tachycardique atriale (1030), et uniquement si aucune activité tachycardiaque atriale intrinsèque n'est présente, l'exécution d'une stimulation de post-traitement atrial (1040), où la stimulation de post-traitement (1040) est étalée sur la plage de 1 minute à 7 jours et est exécutée avec une fréquence de stimulation plus basse que la stimulation anti-tachycardique atriale (1030).

**2.** Ensemble implantable selon la revendication 1, dans lequel le programme fait en sorte que le processeur (950), après l'exécution de la stimulation anti-tachycardique atriale (1030), vérifie tout d'abord, au moyen du dispositif de détection (920) si la tachycardie atriale est terminée et n'effectue alors la stimulation de post-traitement atriale (1040) que si la fin d'une tachycardie atriale a été constatée.

**3.** Ensemble implantable selon la revendication 1 ou la revendication 2, dans lequel le programme fait en sorte que le processeur (950) administre la stimulation de post-traitement atrial (1040) avec une fréquence de stimulation inférieure ou égale à 200 bpm, 180 bpm, 150 bpm, 140 bpm, 130 bpm, 120 bpm, 110 bpm, ou de 100 bpm.

**4.** Ensemble implantable selon l'une des revendications précédentes, dans lequel le programme fait en sorte que le processeur (950) exécute la stimulation de post-traitement atriale (1040) sous forme d'une stimulation surmultipliée atriale droite.

5. Ensemble implantable selon l'une des revendications 1 à 3, dans lequel le programme fait en sorte que le processeur (950) exécute la stimulation de post-traitement atriale (1040) sous forme d'une stimulation surmultipliée atriale gauche.

6. Ensemble implantable selon l'une des revendications 1 à 3, dans lequel le programme fait en sorte que le processeur (950) exécute la stimulation de post-traitement atriale (1040) sous forme d'une stimulation surmultipliée bi-atriale.

7. Ensemble implantable selon l'une des revendications précédentes, dans lequel le programme fait en sorte que le processeur (950) exécute la stimulation de post-traitement atriale (1040) pendant un premier intervalle de temps.

8. Ensemble implantable selon l'une des revendications précédentes, dans lequel le programme fait en sorte que le processeur (950) exécute la stimulation de post-traitement atriale (1040) pendant un nombre défini de cycles cardiaques.

9. Ensemble implantable selon l'une des revendications précédentes, dans lequel le programme fait en sorte que le processeur (950) exécute la stimulation de post-traitement atriale (1040) de telle manière que la pression dans l'oreillette gauche et/ou droite est diminuée au moins sur une courte durée, de sorte que le risque d'une régurgitation de valve mitrale est réduite ou empêchée au moins sur une courte durée, de sorte que la précharge du coeur est réduite au moins sur une courte durée, de sorte que la pression sanguine d'un patient dont le coeur est stimulé par l'ensemble est réduite au moins sur une courte durée, et/ou de sorte qu'un bilan en oxygène myocardique est amélioré au moins sur une courte durée en ce qu'un délai AV déterminé comme optimal pour le patient, et/ou une stimulation biventriculaire, et/ou une fréquence de stimulation ventriculaire augmentée est réglé(e).

10. Ensemble implantable selon l'une des revendications précédentes, dans lequel le dispositif de stimulation atriale (930) est conçu pour exécuter la stimulation anti-tachycardique atriale (1030) et/ou la stimulation de post-traitement atriale (1040) sous forme d'une stimulation électrique ou d'une stimulation optique.

11. Produit-programme informatique avec un code lisible par ordinateur qui fait en sorte qu'un processeur (950) exécute les étapes suivantes, lorsqu'il est exécuté sur le processeur (950) :

    a) la détection (1010), au moyen d'un dispositif de détection (920), s'il y a une tachycardie atriale cardiaque à traiter dans un coeur humain ou animal,

    b) si une tachycardie atriale à traiter est présente, l'exécution d'une stimulation anti-tachycardique atriale (1030) au moyen du dispositif de stimulation atriale (920),

    c) après l'exécution de la stimulation anti-tachycardique atriale (1030), et uniquement si aucune activité tachycardiaque atriale intrinsèque n'est présente, l'exécution d'une stimulation de post-traitement atriale (1040), où la stimulation de post-traitement (1040) est étalée sur la plage de 1 minute à 7 jours et est exécutée avec une fréquence de stimulation plus basse que la stimulation anti-tachycardique atriale (1030).

FIG 1

FIG 2

EP 3 733 240 B1

FIG 3

FIG 4

34

FIG 5

FIG 6

FIG 7

FIG 8

940

950

960

920

930

910

FIG 9

900

1010

1020

n

y

1030

1040

FIG 10

FIG 11

FIG 12

FIG 13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6876880 B2
- US 7107098 B2
- US 7280869 B2

- US 8457739 B2
- EP 2077136 A2
- WO 2006105140 A2